(19)

Europäisches Patentamt

European Patent Office

Office européen des brevets

(11) **EP 3 192 435 A2**

(12) **EUROPEAN PATENT APPLICATION**

(43) Date of publication:
19.07.2017 Bulletin 2017/29

(51) Int Cl.:
*A61B 5/00* (2006.01)     *A61B 8/08* (2006.01)

(21) Application number: **16205319.3**

(22) Date of filing: **20.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.12.2015   JP 2015254371**
**12.05.2016   JP 2016096093**

(71) Applicant: **Canon Kabushiki Kaisha**
**Tokyo 146-8501 (JP)**

(72) Inventors:
• **NANAUMI, Ryuichi**
**Ohta-ku, Tokyo 146-8501 (JP)**
• **FUKUTANI, Kazuhiko**
**Ohta-ku, Tokyo 146-8501 (JP)**
• **FURUKAWA, Yukio**
**Ohta-ku, Tokyo 146-8501 (JP)**
• **NAKAMURA, Yoshiko**
**Ohta-ku, Tokyo 146-8501 (JP)**

(74) Representative: **Walker, Philip Martin**
**Canon Europe Ltd**
**European Patent Department**
**3 The Square**
**Stockley Park**
**Uxbridge, Middlesex UB11 1ET (GB)**

(54) **INFORMATION ACQUISITION APPARATUS, SIGNAL PROCESSING METHOD, AND PROGRAM**

(57)     There is provided an information acquisition apparatus configured to process a signal acquired by receiving an acoustic wave generated in a subject by irradiating the subject with light and propagated through a medium provided between the subject and a reception means. The information acquisition apparatus includes a position setting means configured to set a position of interest, and an acquisition means configured to determine a first signal of interest derived from the acoustic wave generated at the position of interest and propagated through the medium as a transverse wave from among the signals with use of information indicating an acoustic velocity of the transverse wave in the medium, and acquire subject information at the position of interest with use of the first signal of interest.

# FIG.5A

EP 3 192 435 A2

# FIG.5B

# FIG.5C

# FIG.5D

# FIG.5E

**Description**

BACKGROUND OF THE INVENTION

Field of the Invention

The present invention relates to an information acquisition apparatus configured to acquire subject information with use of a signal derived from an acoustic wave.

Description of the Related Art

There is proposed an information acquisition apparatus such as a photoacoustic imaging apparatus and an ultrasonographic imaging apparatus, as a technique for acquiring information inside a subject such as a living body by receiving an acoustic wave.

United States Patent No. 6607489 discusses an ultrasonic imaging apparatus using a compression plate that compresses a breast. United States Patent No. 6607489 discusses that a delay time of an ultrasonic wave is calculated while refraction of the ultrasonic wave due to the compression plate is taken into consideration. United States Patent No. 6607489 discusses that the refraction of the ultrasonic wave due to the compression plate is calculated according to Snell's law. More specifically, United States Patent No. 6607489 discusses that the delay time is calculated while refraction of a longitudinal wave according to Snell's law and propagation thereof through the compression plate as the longitudinal wave are taken into consideration.

However, in a case where there are the subject and a medium in a path along which the acoustic wave is propagated, the subject information may be acquired with lower accuracy due to an influence other than the refraction of the longitudinal wave according to Snell's law.

In a case where there is a solid medium in a path along which an acoustic wave is propagated, a part of a longitudinal wave, which is the acoustic wave, may be converted into a transverse wave on a surface of this medium. For example, in a case where a holding unit that holds a living body is a solid object, when the acoustic wave in the form of the longitudinal wave propagated in the living body reaches the surface of the holding unit with an incident angle generated therebetween, a part thereof is converted into the transverse wave. Then, when the transverse wave is propagated in the holding unit and reaches a medium on a reception side where a reception unit for the acoustic wave is located, the transverse wave is reconverted into the longitudinal wave. At this time, an acoustic velocity is different between the longitudinal wave and the transverse wave. Therefore, a propagation time taken until arrival at the reception unit is different between the acoustic wave propagated to the reception unit as the longitudinal wave from beginning to end and the acoustic wave propagated to the reception unit after being converted into the transverse wave temporarily. Further, an acoustic ray of an acoustic wave refracted by the holding unit travels along a different path between the longitudinal wave and the transverse wave. This difference in the path also affects the propagation time.

Regarding this phenomenon, United States Patent No. 6607489 fails to discuss an influence due to the conversion from the longitudinal wave into the transverse wave. Therefore, the method discussed in United States Patent No. 6607489, in which the conversion from the longitudinal wave into the transverse wave is not taken into consideration, leads to deterioration of accuracy of acquired subject information (deterioration of an image quality). More specifically, the deterioration of the image quality refers to, for example, deterioration of an image quality of a resolution and a contrast of an image.

The present invention is directed to an information acquisition apparatus capable of acquiring the subject information with high accuracy even when there is a different medium from the subject in the path along which the acoustic wave is propagated.

SUMMARY OF THE INVENTION

According to a first aspect of the present invention, there is provided information acquisition apparatus as specified in claims 1 to 13. According to a second aspect of the present invention, there is provided a signal processing method as specified in clam 14. According to a third aspect of the present invention, there is provided a program as specified in clam 15.

Further features of the present invention will become apparent from the following description of embodiments with reference to the attached drawings. Similar components will be identified by similar reference numerals in principle, and redundant descriptions thereof will be omitted.

BRIEF DESCRIPTION OF THE DRAWINGS

Fig. 1 is a schematic diagram of an information acquisition apparatus according to a first embodiment.
Figs. 2A, 2B, 2C, and 2D illustrate details of a probe according to the first embodiment.
Fig. 3 is a block diagram illustrating a configuration around a computer according to the first embodiment.
Fig. 4 is a flowchart illustrating a method for acquiring subject information according to the first embodiment.
Figs. 5A, 5B, 5C, 5D, and 5E illustrate refraction of an acoustic ray.
Figs. 6A and 6B illustrate a planar approximation of a holding cup when the acoustic ray is calculated according to the first embodiment.
Fig. 7 is a schematic diagram of an information acquisition apparatus using a holding unit configured as parallel flat plates.
Fig. 8 illustrates a straight-line approximation of the acoustic ray when the acoustic ray is calculated according to the first embodiment.
Fig. 9 illustrates a relationship between an incident angle and each of pressure transmittances of a longitudinal wave and a transverse wave.
Figs. 10A and 10B illustrate a graphical user interface (GUI) according to the first embodiment.
Figs. 11A and 11B illustrate a straight-line approximation of the acoustic ray when the acoustic ray is calculated according to a second embodiment.
Fig. 12 illustrates a relationship between each of the pressure transmittances and a hypothetical critical angle.
Figs. 13A and 13B illustrate a flow for acquiring the hypothetical critical angle.
Fig. 14 illustrates a calculation model used in an example.
Fig. 15 illustrates a result of a simulation according to the example.
Figs. 16A, 16B, and 16C illustrate a table storing a propagation time.
Fig. 17 is a schematic diagram of an information acquisition apparatus according to a fifth embodiment.
Fig. 18 is a flowchart illustrating a method for acquiring the subject information according to the fifth embodiment.
Fig. 19 illustrates respective acoustic rays of transmission and reception according to the fifth embodiment.
Fig. 20 is a schematic diagram of an information acquisition apparatus according to a sixth embodiment.
Fig. 21 is a flowchart illustrating a method for acquiring the subject information according to the sixth embodiment.
Fig. 22 illustrates respective acoustic rays of transmission and reception according to the sixth embodiment.

DESCRIPTION OF THE EMBODIMENTS

<Configuration of Information Acquisition Apparatus>

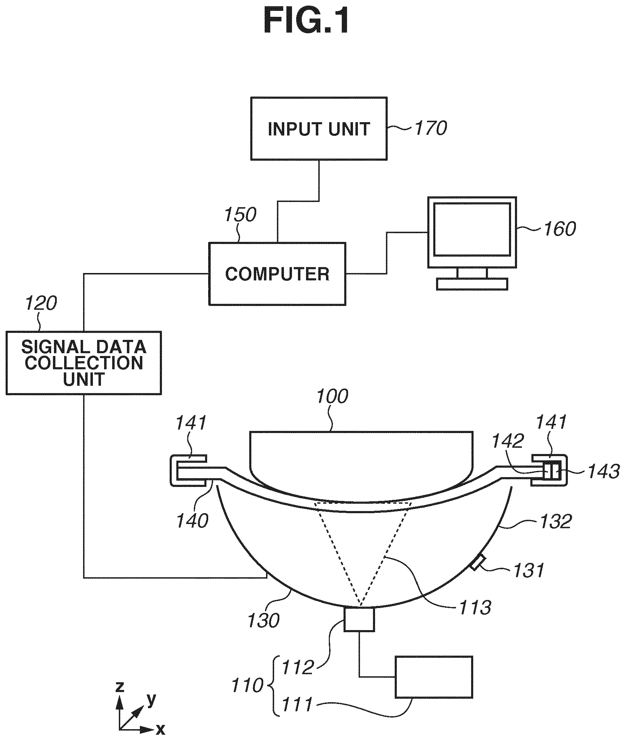

Fig. 1 is a schematic diagram of an information acquisition apparatus according to a first embodiment. Now, each of components of the apparatus will be described. The information acquisition apparatus illustrated in Fig. 1 includes a light irradiation unit 110, a probe 130, a holding cup 140, a signal data collection unit 120, a computer 150, a display unit 160, and an input unit 170. A measurement target is a subject 100.

The light irradiation unit 110 irradiates the subject 100 with pulsed light 113, and an acoustic wave is generated in the subject 100. The acoustic wave generated due to light with the aid of the photoacoustic effect is also called a photoacoustic wave. The probe 130 outputs an electric signal as an analog signal by receiving the photoacoustic wave. The signal data collection unit 120 converts the electric signal as the analog signal output from the probe 130 into a digital signal, and outputs the converted signal to the computer 150. The computer 150 stores the digital signal output from the signal data collection unit 120 as signal data derived from the photoacoustic wave.

The computer 150 generates image data indicating information regarding the subject 100 (subject information) by performing signal processing on the stored digital signal. Further, the computer 150 outputs the image data to the display unit 160 after performing image processing on the acquired image data. An image of the information regarding the subject 100 is displayed on the display unit 160. A doctor as a user can make a diagnosis by confirming the image of the information regarding the subject 100 that is displayed on the display unit 160.

The subject information acquired by the photoacoustic apparatus according to the present embodiment is at least one of a generated acoustic pressure of the photoacoustic wave (initial acoustic pressure), a density of optical absorption energy, an optical absorption coefficient, and information regarding a concentration of a substance forming the subject 100. The information regarding the concentration of the substance is a concentration of oxyhemoglobin, a concentration of deoxyhemoglobin, a total concentration of hemoglobin, oxygen saturation, or the like. The total concentration of hemoglobin corresponds to a sum of the concentration of oxyhemoglobin and the concentration of deoxyhemoglobin. The oxygen saturation corresponds to a ratio of oxyhemoglobin to a total amount of hemoglobin. The photoacoustic apparatus according to the present embodiment acquires image data indicating a value of the above-described information at each position (each position in a two-dimensional or three-dimensional space) in the subject 100.

Now, each of the units of the information acquisition apparatus according to the present embodiment will be described below in detail.

<Light Irradiation Unit 110>

The light irradiation unit 110 includes a light source 111, which generates the pulsed light 113, and an optical system 112, which guides the pulsed light 113 emitted from the light source 111 to the subject 100.

A pulse width of the light 113 generated by the light source 111 may be a pulse width of 1 ns or wider and 100 ns or narrower. Further, a wavelength of the light 113 may be a wavelength that falls within a range from about 400 nm to 1600 nm. In a case where a blood vessel located close to a surface of a living body is imaged at a high resolution, it is desirable to use a wavelength largely absorbable by the blood vessel (400 nm or longer and 700 nm or shorter). On the other hand, in a case where a deep portion in the living body is imaged, desirably, the information acquisition apparatus may use light having a wavelength (700 nm or longer and 1100 nm or shorter) typically little absorbable at a background tissue of the living body (e.g., water and fat).

A laser or a light-emitting diode can be used as the light source 111. Further, in a case where the subject 100 is measured with use of light beams having a plurality of wavelengths, the light source 111 may be a light source capable of converting the wavelength. Another possible configuration in the case where the subject 100 is irradiated with the light of plurality of wavelengths is to prepare a plurality of light sources that respectively generates light beams having wavelengths different from one another, and irradiate the subject 100 from each of the light sources alternately or in turn. Even when the plurality of light sources is used, they will be collectively expressed as the light source. Various types of lasers, such as a solid-state laser, a gas laser, a dye laser, and a semiconductor laser, can be used as the laser. For example, a pulse laser, such as a neodymium-doped yttrium aluminum garnet (Nd:YAG) laser and an alexandrite laser, may be used as the light source 111. Alternatively, a titanium sapphire (Ti:sa) laser using Nd:YAG laser light as excitation light, or an optical parametric oscillator (OPO) laser may be used as the light source 111. Alternatively, a microwave source may be used as the light source 111.

An optical element such as a lens, a mirror, and an optical fiber can be used as the optical system 112. In a case where a breast is handled as the subject 100, for example, it is desirable to irradiate the subject 100 while expanding a beam diameter of the pulsed light 113, whereby a light emission portion of the optical system 112 may include a diffusing plate or the like that diffuses the light 113. On the other hand, in a case where the information acquisition apparatus functions as a photoacoustic microscope, the light emission portion of the optical system 112 may include a lens or the like to allow the subject 100 to be irradiated while the beam is focused thereon so as to increase a resolution.

The subject 100 may be irradiated with the pulsed light 113 directly from the light source 111 with the light irradiation unit 110 unequipped with the optical system 112.

<Probe 130>

The probe 130 as a reception unit includes a transducer 131, which outputs the electric signal by receiving the acoustic wave, and a support member 132, which supports the transducer 131.

A piezoelectric ceramic material represented by lead zirconate titanate (PZT), a polymer piezoelectric film material represented by polyvinylidene fluoride (PVDF), or the like can be used as a member forming the transducer 131. Alternatively, an element other than the piezoelectric element may be used. For example, an electrostatic capacitance type transducer (a capacitive micro-machined ultrasonic transducer (CMUT)) or a transducer using a Fabry-Perot interferometer can be used. Any transducer may be employed as long as the transducer can output the electric signal by receiving the acoustic wave. Further, the signal acquired by the transducer 131 is a time-resolved signal. In other words, an amplitude of the signal acquired by the reception element indicates a value based on the acoustic pressure (e.g., a value proportional to the acoustic pressure) received by the transducer 131 at each time.

Frequency components forming the photoacoustic wave are typically 100 KHz to 100 MHz, and an element capable of detecting these frequencies can be selected as the transducer 131.

The support member 132 may be made from, for example, a metallic material having high mechanical strength. In a case where the acoustic wave is acquired by driving the support member 132 for a scan, it is desirable to use, for example, a polymer material such as plastic due to its lightness in weight from the viewpoint of a mechanical load. A surface located in a subject direction may be prepared as a mirrored surface or may be processed so as to scatter the light 113 thereon, to allow a large amount of irradiation light to be incident on the subject 100. In the present embodiment, the support member 132 is shaped like a semispherical shell and is configured to be able to support a plurality of transducers 131 on the semispherical shell. In this case, respective directional axes of the transducers 131 arranged on the support member 132 are collected in the vicinity of a center of a curvature of the semi-sphere. Then, an image quality increases at near the center of the curvature when the subject 100 is imaged with use of a group of electric signals output from the plurality of transducers 131. The support member 132 may be configured in any manner as long as the support member 132 can support the transducers 131. The support member 132 may be configured in such a manner that the plurality of transducers 131 is disposed so as to be arrayed on a flat surface or a curved surface like an array called a 1-dimensional (D) array, a 1.5-D array, a 1.75-D array, or a 2-D array.

Further, the support member 132 may function as a container that stores an acoustic matching material therein. In other words, the support member 132 may be used as a container for placing the acoustic matching material between the transducers 131 and the holding cup 140.

Further, the probe 130 may include an amplifier that amplifies the chronological analog signal output from the transducer 131. Further, the probe 130 may include an analog-to-digital (A/D) converter that converts the chronological analog signal output from the transducer 131 into a chronological digital signal. In other words, the probe 130 may include the signal data collection unit 120, which will be described below.

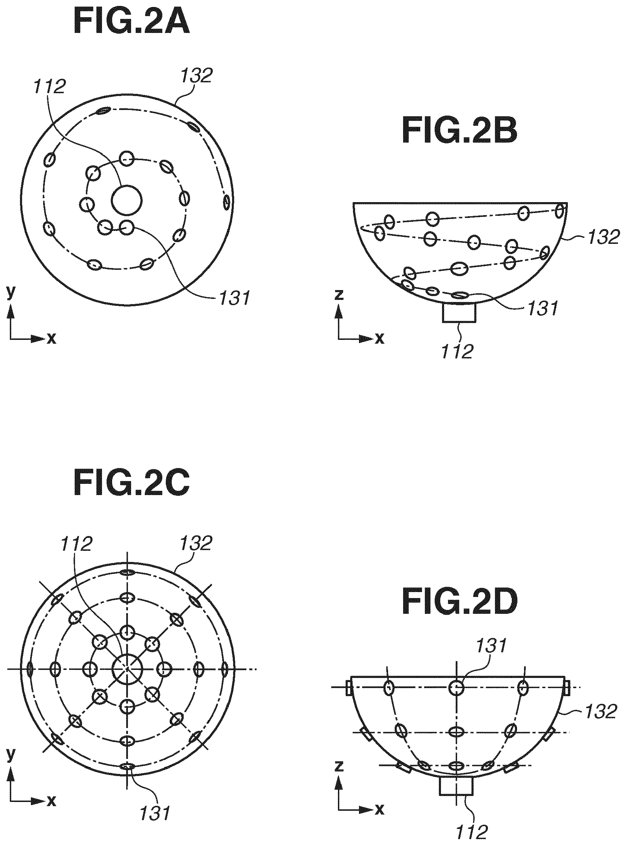

Ideally, it is desirable that the transducers 131 are arranged so as to surround the subject 100 from all around the subject 100 to allow the acoustic wave to be detected from various angles. However, in a case where the transducers 131 cannot be arranged so as to broadly surround the subject 100 from all around the subject 100, the transducers 131 may be arranged on the semispherical support member 132 to make the layout thereof more resemble a state surrounding the subject 100 from all around the subject 100 as illustrated in Fig. 2.

The layout and the number of the transducers 131, and the shape of the support member 132 can be selected differently as long as they are optimized according to the subject 100, and any kind of probe 130 may be employed regarding the present invention.

Figs. 2A, 2B, 2C, and 2D illustrate details of the probe 130. Figs. 2A and 2B illustrate the probe 130 in which the transducers 131 are spirally arranged. Figs. 2C and 2D illustrate the probe 130 in which the transducers 131 are radially arranged. Figs. 2A and 2C illustrate the probe 130 as viewed from a z axis direction in Fig. 1, and Figs. 2B and 2D illustrate the probe 130 as viewed from a y axis direction in Fig. 1. In either case, the transducers 131 are arranged on the spherical surface of the probe 130, and can receive the photoacoustic wave generated in the subject 100 from various angular directions. In Figs. 2A, 2B, 2C, and 2D, the transducers 131 are spirally or radially arranged, but how to lay out the transducers 131 is not limited to these examples. For example, the transducers 131 may be arranged in a lattice pattern on the spherical surface.

For the probe layouts as illustrated in Figs. 2A, 2B, 2C, and 2D, it is desirable that the probe 130 is disposed in such a manner that a reception surface of the probe 130 faces in a direction toward a center of the curved surface of the support member 132. The employment of such a layout causes a focal point of the probe 130 from which the acoustic wave is received (a reception focal point) to be positioned at the center of the curved surface of the support member 132. The acoustic wave generated from around this reception focal point can be measured from multiple directions, so that the contrast and the resolution are improved around the reception focal point. The orientation of the reception surface and the position of the reception focal point of the probe 130 can be arbitrarily selected according to the direction in which the subject 100 is located and the shape of the subject 100, the shape of the holding unit, or a method for driving the support member 132 for the scan.

A space between the probe 130 and the holding cup 140 is filled with a medium that permits the photoacoustic wave to be propagated therethrough. A material employable for this medium is a material that permits the photoacoustic wave to be propagated therethrough, allows acoustic characteristics to match each other at an interface between the subject 100 and the transducer 131, and allows the photoacoustic wave to be transmitted therethrough at as a high transmittance as possible. For example, water or an ultrasonic gel can be employed as this medium.

<Holding Cup 140>

The holding cup 140 as the holding unit is used to maintain the shape of the subject 100 during the measurement. Holding the subject 100 with the holding cup 140 can constrain a motion of the subject 100 and keep a position of the subject 100 within the holding cup 140.

The holding cup 140 may be made from a material having hardness sufficient for the holding cup 140 to hold the subject 100. It is desirable that the holding cup 140 is made from a material that permits the light 113 used for the measurement to transmit therethrough. The holding cup 140 may be made from a material having similar impedance to the subject 100. For example, impedance of fat, which is a main composition of a mammary gland, is about 1.38. In this case, the holding cup 140 may be made from a material having impedance of about 1.38. The holding cup 140 may be made from a material having impedance about 0.5 times to twice as high as the subject 100. In a case where an object having a curved surface, such as a breast, is handled as the subject 100, the holding cup 140 may be formed into a concaved shape. In this case, the subject 100 can be inserted in the concaved potion of the holding cup 140. A resin material, such as polycarbonate, polyethylene, and polyethylene terephthalate, can be used as the material of the holding cup 140.

In the case where the reception focal point for the acoustic wave is positioned according to the orientation of the reception surface of the probe 130, it is desirable to appropriately select the shape of the holding cup 140 so as to allow the holding cup 140 to keep the subject 100 at the reception focal point. In the case where the support member 132 has the probe layout illustrated in Fig. 2, the shape of the holding cup 140 may be formed into a concaved shape, and the holding cup 140 may be disposed so that the subject 100 is placed closer to the position of the reception focal point.

The holding cup 140 is mounted on a mounting unit 141. The mounting unit 141 may be configured to allow the holding cup 140 to be replaced among a plurality of types of holding cups according to the size of the subject 100. For example, the mounting unit 141 may be configured to allow the holding cup 140 to be replaced among holding cups having different curvature radii and different curvature centers.

Further, a tag 142, on which specifications of the holding cup 140 are registered, may be set at the holding cup 140. For example, the specifications of the holding cup 140, such as the radius of the curvature, the center of the curvature, an acoustic velocity of a longitudinal wave, an acoustic velocity of a transverse wave, and an identification (ID) for identifying the holding cup 140, can be registered on the tag 142. The specifications registered on the tag 142 are read out by a readout unit 143, and transferred to the computer 150. The readout unit 143 may be set at the mounting unit 141 so as to facilitate the readout of the tag 142 when the holding cup 140 is mounted on the mounting unit 141. For example, the tag 142 is a barcode, and the readout unit 143 is a barcode reader.

<Signal Data Collection Unit 120>

The signal data collection unit 120 includes an amplifier that amplifies the electric signal that is the analog signal output from the transducer 131, and an A/D converter that converts the analog signal output from the amplifier into the digital signal. The signal data collection unit 120 may be embodied with use of a field programmable gate array (FPGA) chip or the like. The digital signal output from the signal data collection unit 120 is stored into a storage unit in the computer 150. The signal data collection unit 120 is also called a data acquisition system (DAS). The electric signal herein is a concept including both the analog signal and the digital signal. The signal data collection unit 120 may be connected to a photo-detection sensor mounted at the light emission portion of the light irradiation unit 110, and processing thereof may be started synchronously by being triggered by the emission of the pulsed light 113 from the light irradiation unit 110.

<Computer 150>

The computer 150 includes a processing unit, the storage unit, and a control unit. A function of each of the units will be described when a processing flow is described.

The storage unit can be embodied with use of a non-transitory storage medium, such as a read only memory (ROM), a magnetic disk, and a flash memory. Alternatively, the storage unit may be a volatile medium, such as a random access memory (RAM). A storage medium storing a program is a non-transitory storage medium.

A unit in charge of a calculation function as the processing unit can be embodied with use of a processor, such as a central processing unit (CPU) and a graphics processing unit (GPU), or a calculation circuit, such as an FPGA chip. Not only these units may be embodied with use of a single processor or calculation circuit, but also these units may be embodied with use of a plurality of processors and/or calculation circuits. The processing unit may process the reception signal while receiving various kinds of parameters, such as an acoustic velocity in the subject 100 and the configuration of the holding cup 140, from the input unit 170.

The control unit is embodied with use of a calculation element, such as a CPU. The control unit controls an operation of each of the units of the photoacoustic apparatus. The control unit may control each of the units of the photoacoustic apparatus while receiving an instruction signal issued according to various kinds of operations, such as a start of the measurement, from the input unit 170. Further, the control unit reads out a program code stored in the storage unit, and controls activation of each of the units of the photoacoustic apparatus.

The computer 150 may be a workstation designed specifically therefor. Further, each of the units of the computer 150 may be embodied with use of a different hardware device. Alternatively, at least a part of the units of the computer 150 may be embodied with use of a single hardware device.

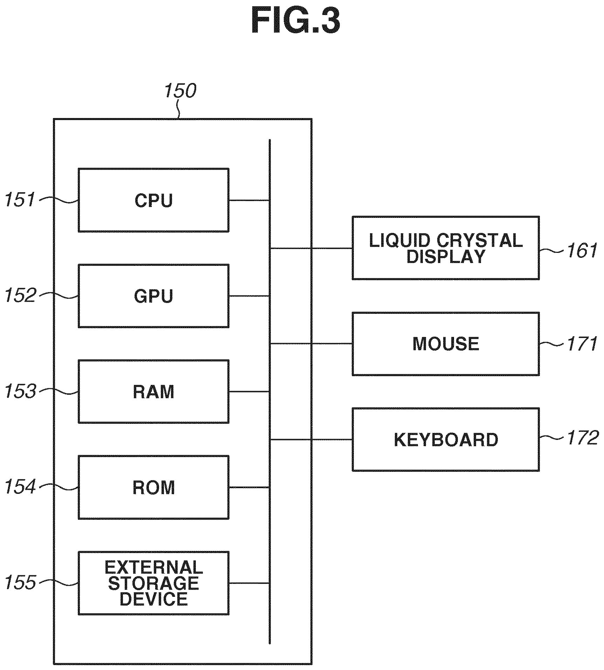

Fig. 3 is a block diagram illustrating a specific configuration of the computer 150 according to the present embodiment. The computer 150 according to the present embodiment includes a CPU 151, a GPU 152, a RAM 153, a ROM 154, and an external storage device 155. Further, a liquid crystal display 161 as the display unit 160, and a mouse 171 and a keyboard 172 as the input unit 170 are connected to the computer 150.

Further, the computer 150 and the plurality of transducers 131 may be provided in such a configuration that they are contained in a common casing. However, the information acquisition apparatus may also be configured in such a manner that a computer contained in the casing performs a part of the signal processing, and a computer provided outside the casing performs the rest of the signal processing. In this case, the computers provided inside and outside the casing can be collectively referred to as the computer 150 according to the present embodiment.

<Display Unit 160>

The display unit 160 is a display, such as a liquid crystal display and an organic electro luminescence (EL) display. The display unit 160 is a device that displays, for example, the image based on the subject information, and a numerical value at a specific position acquired by the computer 150. The display unit 160 may display a graphical user interface (GUI) for operating the image and the apparatus. When the subject information is displayed, the subject information can also be displayed after image processing (an adjustment of a luminance value and/or the like) by the display unit 160 or the computer 150.

<Input Unit 170>

The input unit 170 can include a mouse, a keyboard, operable by the user. Further, the display unit 160 may be constructed with use of a touch panel, thereby allowing the display unit 160 to serve as the input unit 170.
Each of the units of the photoacoustic apparatus may be configured as an individually separate device, or may be configured as an integrated single device. Alternatively, at least a part of the units of the photoacoustic apparatus may be configured as an integrated single device.

<Subject 100>

The subject 100 will be described below, although subject 100 is not a component forming the photoacoustic apparatus. The photoacoustic apparatus according to the present embodiment can be used for the purpose of a diagnosis, follow-up monitoring during or after a chemical treatment, or the like of a malignant tumor, a vascular disease, or the like of a human or an animal. Therefore, a site targeted for the diagnosis, such as a living body, in particular, a breast, a neck, an abdomen, or four limbs including fingers and toes of a human or an animal, is expected to be handled as the subject 100. For example, in the case where the measurement target is a human body, oxyhemoglobin, deoxyhemoglobin, a blood vessel including a large amount of them, a new blood vessel formed close to a tumor, or the like may be handled as an optical absorber target. Alternatively, for example, plaque of a carotid artery wall may be handled as the optical absorber target. Alternatively, an externally introduced substance, such as a dye (e.g., methylene blue (MB) and indocyanine green (ICG)), gold fine particles, or a substance acquired by collecting or chemically modifying them, may be handed as the optical absorber.

<Method for Acquiring Subject Information>

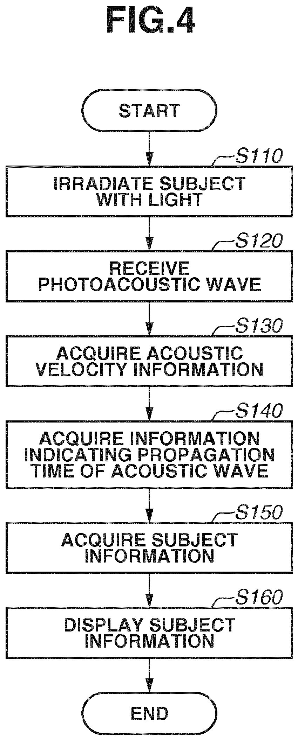

Next, each of processes in a method for acquiring the subject information according to the present embodiment will be described with reference to Fig. 4. The computer 150 controls the operation of each of the units of the information acquisition apparatus, by which each of the processes is performed.

<Step S110: Process for Irradiating Subject with Light>

The subject 100 is irradiated with the light generated by the light source 111 as the pulsed light 113 via the optical system 112. Then, the pulsed light 113 is absorbed inside the subject 100, and the photoacoustic wave is generated due to the photoacoustic effect.

(Step S120: Process for Receiving Photoacoustic Wave)

In the present process, the probe 130 receives the photoacoustic wave, and the electric signal is output from the transducer 131. The output reception signal is transferred to the computer 150.

<Step S130: Process for Acquiring Acoustic Velocity Information>

The computer 150 acquires information indicating an acoustic velocity $c_1$ of a longitudinal wave in the subject 100, an acoustic velocity $c_2^l$ of a longitudinal wave in the holding cup 140, and an acoustic velocity $c_2^t$ of a transverse wave in the holding cup 140.
The information indicating the acoustic velocity $c_2^l$ of the longitudinal wave and the information indicating the acoustic velocity $c_2^t$ of the transverse wave in the holding cup 140 may be acquired in such a manner that premeasured data is stored in the storage unit in advance, and the computer 150 reads out the stored premeasured data from the storage unit in the present process. Further or alternatively, an equation or a table that indicates a relationship of each of the acoustic velocity $c_2^l$ of the longitudinal wave and the acoustic velocity $c_2^t$ of the transverse wave in the holding cup 140 with respect to a temperature of the holding cup 140 may be stored in the storage unit in advance. Then, in this process, a temperature measurement unit may measure the temperature of the holding cup 140, and the computer 150 may acquire the acoustic velocities $c_2^l$ and $c_2^t$ corresponding to the measured temperature according to the equation or the table indicating the relationship.

Alternatively, the acoustic velocities $c_2^l$ and $c_2^t$ may be acquired in such a manner that the user inputs the acoustic velocities $c_2^l$ and $c_2^t$ in the holding cup 140 via the input unit 170, and the computer 150 receives this information.

Alternatively, the computer 150 may acquire the acoustic velocities $c_2^l$ and $c_2^t$ in the holding cup 140 that correspond to the holding cup 140 mounted on the mounting unit 141. For example, the readout unit 143 may read out the information indicating the acoustic velocities $c_2^l$ and $c_2^t$ in the holding cup 140 that is registered on the tag 142 attached to the holding cup 140, and transfer the read information to the computer 150. The computer 150 may acquire the information indicating the acoustic velocities $c_2^l$ and $c_2^t$ in the holding cup 140 that is read out by the readout unit 143. Alternatively, the acoustic velocities $c_2^l$ and $c_2^t$ in the holding cup 140 may be acquired in such a manner that the user inputs the ID for identifying the holding cup 140 that is allocated to the holding cup 140 via the input unit 170, and the computer 150 reads out the acoustic velocities $c_2^l$ and $c_2^t$ in the holding cup 140 that correspond to the input ID for identifying the holding cup 140 from the storage unit.

The acoustic velocity $c_1$ of the longitudinal wave in the subject 100 may also be acquired by a similar method to the method for acquiring the acoustic velocity $c_2^l$ of the longitudinal wave and the acoustic velocity $c_2^t$ of the transverse wave in the holding cup 140. However, the acoustic velocity $c_1$ of the longitudinal wave in the subject 100 is different for each subject 100. Therefore, it is desirable to acquire new data for each subject 100. The computer 150 may acquire the acoustic velocity $c_1$ in the subject 100 with use of the signal derived from the acoustic wave generated from the subject 100. According to this method, an acoustic velocity specific to each subject 100 can be acquired without requiring an increase in the scale of the apparatus. The computer 150 may acquire the acoustic velocity $c_1$ in the subject 100 by another known method.

In the above description, the process for acquiring the acoustic velocity has been described based on the example in which the computer 150 acquires the acoustic velocity itself, but, in the present process, any parameter may be acquired as long as the acquired parameter is a parameter that allows the acoustic velocity to be estimated therefrom. For example, the acoustic velocity can be calculated from a density $\rho$ and a volume elasticity modulus K, whereby the density $\rho$ and the volume elasticity modulus K may be acquired and the acoustic velocity may be estimated from these parameters in the present process. Herein, the acoustic velocity information also includes these parameters that allow the acoustic velocity to be estimated therefrom, besides a velocity at which the longitudinal wave or the transverse wave is propagated (the acoustic velocity).

<Step S140: Process for Calculating Propagation Time of Acoustic Wave>

In the photoacoustic imaging, the universal back-projection (UBP) method expressed by, for example, an equation (1) can be used as a method for calculating the initial acoustic pressure.

$$p_0(\mathbf{r}_0) = \frac{\sum_i^N b\left(\mathbf{r}_i, t = \frac{|\mathbf{r}_i - \mathbf{r}_0|}{c}\right) \cdot \Delta\Omega_i}{\sum_i^N \Delta\Omega_i}$$

$$b(\mathbf{r}, t) = 2p(\mathbf{r}, t) - 2t\frac{\partial p(\mathbf{r}, t)}{\partial t} \qquad (1)$$

In this equation (1), $r_0$ represents a positional vector indicating a position to be reconstructed (also referred to as a reconstruction position or a position of interest), $p_0(r_0, t)$ represents an initial acoustic pressure at the position to be reconstructed, and c represents an acoustic velocity in the propagation path. Further, $\Delta\Omega_i$ represents a solid angle of an i-th transducer 131 as viewed from the position to be reconstructed, and N represents the number of transducers 131 for use in the reconstruction. The equation (1) indicates that reception signals $p(r_i, t)$ are subjected to processing such as differentiation, and are subjected to phasing and summing with each of them multiplied by a weight according to the solid angle (back-projection). In the equation (1), t represents a time period during which the photoacoustic wave is propagated along an acoustic ray connecting the position of interest and the transducer 131 to each other (the propagation time). Other calculation processing may be performed in the calculation of $b(r_i, t)$. Examples of the other calculation processing include frequency filtering (e.g., low-pass filtering, high-pass filtering or band-pass filtering), deconvolution, envelop detection, and wavelet filtering. Further, in the present invention, any kind of reconstruction algorithm may be used as long as this algorithm is a method that reconstructs the image by acquiring the propagation time t along the acoustic ray connecting the transducer 131 and the position of interest to each other. For example, filtered back-projection may be employed as the back-projection method in the time domain.

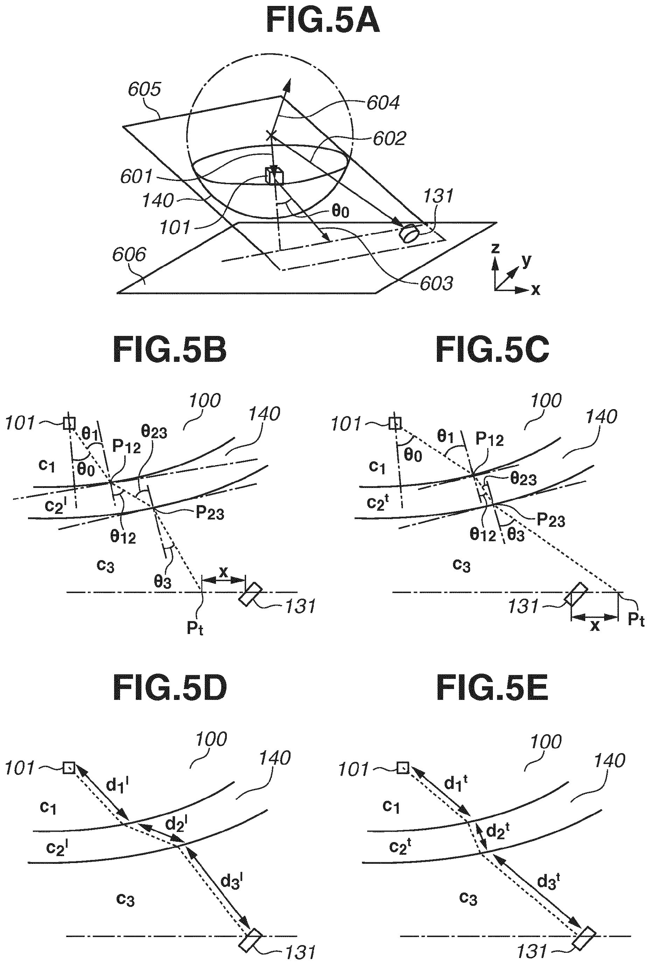

In the present process, the computer 150 calculates two propagation times $t^l$ and $t^t$ for use in the back-projection method in the time domain. Then, $t^l$ represents a propagation time according to an acoustic ray when the acoustic wave generated at the reconstruction position is propagated as the longitudinal wave in the holding cup 140 (a second propagation time). Further, $t^t$ represents a propagation time according to an acoustic ray when the acoustic wave generated at the reconstruction position is propagated as the transverse wave in the holding cup 140 (a first propagation time). A method for calculating them will be described with reference to Figs. 5A, 5B, 5C, 5D, and 5E.

In Fig. 5A, a voxel 101 is a voxel to be reconstructed (also referred to the reconstruction position or the position of interest), a vector 601 is a vector connecting the center of the curvature of the holding cup 140 and the voxel 101 to each other, and a vector 602 is a vector connecting the same center of the curvature and the transducer 131 to each other. A plane 606 is a plane extending in parallel with an xy plane, and having the same z coordinate as the transducer 131. A plane 605 is a plane passing through all of the voxel 101, the transducer 131, and the center of the curvature of the holding cup 140. A vector 603 is a vector located on the plane 605 and extending from the voxel 101 as a start point to a point on the plane 606 as an end point. The computer 150 as a position setting unit sets the position of the voxel 101 (the reconstruction position), the position of the holding cup 140, and the position of the transducer 131 on a calculation area. Then, the computer 150 calculates such an acoustic ray that the end point of the vector 603 or an end point of a line segment formed by refracting the vector 603 reaches coordinates of the transducer 131. The computer 150 acquires the propagation times $t^l$ and $t^t$ by calculating them along this acoustic ray. The method that traces the propagation path along the acoustic ray and calculates a phase delay and the propagation time of the acoustic wave in this manner is called a ray tracing method.

1. Method for Calculating Propagation Time while Taking Refraction into Consideration

First, a method for calculating the propagation times $t^l$ and $t^t$ when refraction due to the holding member is taken into consideration will be described. Figs. 5B and 5C illustrate the plane 605 as viewed from above. Fig. 5B illustrates an acoustic ray of the acoustic wave when the acoustic wave is propagated in the holding cup 140 as the longitudinal wave (second acoustic ray). Fig. 5C illustrates an acoustic ray of the acoustic wave when the acoustic wave is propagated in the holding cup 140 as the transverse wave (first acoustic ray).

First, a straight line is drawn from the voxel 101 toward a surface of the holding cup 140 that faces the subject 100 at an arbitrary angle $\theta_r$, and coordinates of a point $P_{12}$ where the straight line intersects with the holding cup 140 are acquired. The coordinates of the intersection point $P_{12}$ are acquired by, for example, acquiring a vector 604 from a calculation of an outer product of the vector 601 and the vector 602, and calculating a unit vector acquired by rotating the vector 601 by $\theta_r$ around this vector 604 as a rotational axis. A four-dimensional number (quaternion), an affine transformation, an Euler angle, or the like can be used for the calculation of the rotation. The coordinates of the intersection point $P_{12}$ can be acquired with use of the thus-acquired unit vector component, the coordinates of the voxel 101, and the specifications (shape data) of the holding cup 140.

Next, coordinates of an intersection point $P_{23}$ are acquired. Snell's law is applied to a tangent plane (tangent line in Figs. 5B and 5C) of the holding cup 140 at the intersection point $P_{12}$, by which $\theta_{12}$ is acquired. An equation (2) is used if the propagation time $t^l$ of the longitudinal wave is acquired in Fig. 5B, and an equation (3) is used if the propagation time $t^t$ of the transverse wave is acquired in Fig. 5C. In the equations (2) and (3), $c_1$ represents the acoustic velocity in the subject 100, $c_2^l$ represents the acoustic velocity of the longitudinal wave in the holding cup 140, and $c_2^t$ represents the acoustic velocity of the transverse wave in the holding cup 140. Then, $\theta_1$ can be acquired from an inner product of a normal vector of the holding cup 140 at the intersection point $P_{12}$ and the unit vector acquired by rotating the vector 601 by $\theta_r$.

$$\frac{\sin(\theta_1)}{c_1} = \frac{\sin(\theta_{12})}{c_2^l} \qquad (2)$$

$$\frac{\sin(\theta_1)}{c_1} = \frac{\sin(\theta_{12})}{c_2^t} \qquad (3)$$

The coordinates of the intersection point $P_{23}$ can be calculated with use of a unit vector acquired by rotating the vector 601 by $\theta_{12}$ around the vector 604 as a rotational axis, the coordinates of the intersection point $P_{12}$, and the specifications (shape data) of the holding cup 140.

Next, coordinates of an intersection point $P_t$ are acquired. Snell's law is applied to a tangent line of the holding cup 140 at the intersection point $P_{23}$, by which $\theta_3$ is acquired. An equation (4) is used if the propagation time $t^l$ of the longitudinal wave is acquired in Fig. 5B, and an equation (5) is used if the propagation time $t^t$ of the transverse wave is acquired in Fig. 5C. In the equations (4) and (5), $c_3$ represents the acoustic velocity in the medium filling the space between the probe 130 and the holding cup 140. An angle $\theta_{23}$ can be acquired from an inner vector of a normal vector of the holding cup 140 at the intersection point $P_{23}$ and the unit vector acquired by rotating the vector 601 by $\theta_{12}$.

$$\frac{\sin(\theta_{23})}{c_2^l} = \frac{\sin(\theta_3)}{c_3} \qquad (4)$$

$$\frac{\sin(\theta_{23})}{c_2^t} = \frac{\sin(\theta_3)}{c_3} \qquad (5)$$

The coordinates of the intersection point $P_t$ can be calculated with use of a unit vector acquired by rotating the vector 601 by $\theta_3$ around the vector 604 as a rotational axis, the coordinates of the intersection point $P_{23}$, and the z coordinate of the transducer 131.

The above-described calculation is repeated while $\theta_r$ is being changed until the intersection point $P_t$ coincides with the position of the transducer 131, i.e., a distance x between the transducer 131 and the intersection point $P_t$ sufficiently reduces. The bisection method, the golden section method, or the like can be used for the repeated calculation. Figs. 5D and 5E illustrate the acoustic ray of the longitudinal wave and the acoustic ray of the transverse wave when x sufficiently reduces, respectively, with broken lines. The propagation times $t^l$ and $t^t$ can be acquired with use of an equation (6) and an equation (7), respectively. Distances $d_1^l$, $d_2^l$, and $d_3^l$ (in the case of the longitudinal wave) and distances $d_1^t$, $d_2^t$, and $d_3^t$ (in the case of the transverse wave) when the acoustic wave is propagated through the respective layers can be acquired from the respective acoustic rays (coordinates of the respective intersection points).

$$t^l = \frac{d_1^l}{c_1} + \frac{d_2^l}{c_2^l} + \frac{d_3^l}{c_3} \qquad (6)$$

$$t^t = \frac{d_1^t}{c_1} + \frac{d_2^t}{c_2^t} + \frac{d_3^t}{c_3} \qquad (7)$$

2. Method for Calculating Propagation Time Using Planar Approximation of Boundary

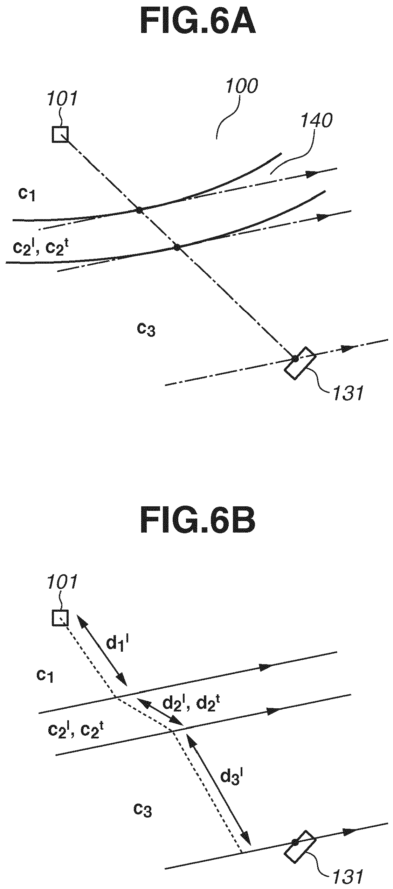

The computer 150 may calculate the propagation times $t^l$ and $t^t$ by approximating a boundary of the holding cup 140 to a plane as illustrated in Fig. 6B. A calculation method in this case will be described.

As illustrated in Fig. 6A, the voxel 101 and the transducer 131 are connected to each other via a straight line, and a point where this straight line intersects with each of boundaries of the holding cup 140 is acquired. A tangent plane (tangent line in Fig. 6A) is acquired at the intersection point on the boundary between the subject 100 and the holding cup 140, and, further, a plane in parallel with this tangent plane is also acquired at the intersection point on the other boundary. The propagation times $t^l$ and $t^t$ are acquired by calculating the equation (2) to the equation (7) based on a calculation model using the planar approximation created in this manner (Fig. 6B). Compared to the method illustrated in Figs. 5A, 5B, 5C, and 5D, due to the approximation of the three-dimensional shape of the holding cup 140 to the two-dimensional shape, the vector calculation such as the calculation of the outer product can be simplified into the geometric calculation on the plane when the coordinates of each of the intersection points are acquired. Further, the angles can be handled as $\theta_r = \theta_1$ and $\theta_{12} = \theta_{23}$. As a result, the calculation is simplified, and a calculation time can be shortened. The calculation model using the planar approximation is effective, for example, when the radius of the curvature of the holding cup 140 is sufficiently large.

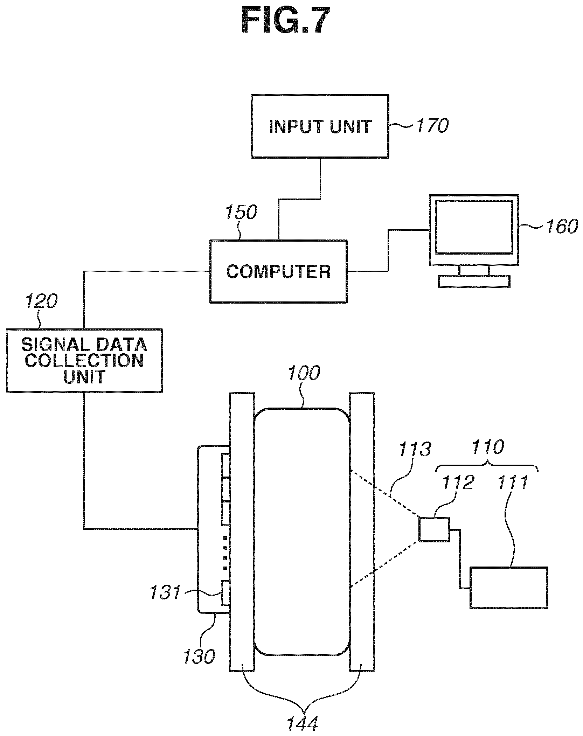

In a case where the subject 100 is held with use of holding plates (parallel flat plates) 144 configured as the holding unit as illustrated in Fig. 7, a difference between the shape of the holding unit and the calculation model using the planar approximation reduces. Therefore, in this case, the computer 150 can correctly calculate the propagation times $t^l$ and $t^t$ by employing the calculation model using the planar approximation. In Fig. 7, the transducers 131 of the probe 130 are arranged on a two-dimensional surface in parallel with the holding plates 144. As long as the holding plates 144 configured as parallel flat plates are employed, the calculation model using the planar approximation can be effectively employed regardless of how the transducers 131 are arrayed. Further, the two holding plates 144 illustrated in Fig. 7 can sandwich and compress the subject 100 therebetween. The holding plates 144 make the subject 100 thinner due to the compression to allow the light 113 to reach as far as the deep portion, thereby succeeding in improvement of the image quality at the deep portion of the subject 100.

3. Method for Calculating Propagation Time Using Straight-line Approximation of Acoustic Ray

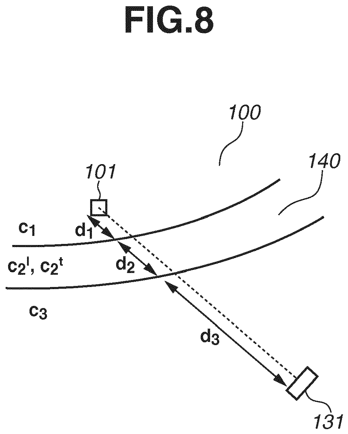

The computer 150 may calculate the propagation times $t^l$ and $t^t$, assuming that the acoustic ray is a straight line as illustrated in Fig. 8. This method is a method in which the voxel 101 and the transducer 131 are connected to each other via a straight line, and this straight line is handled as the acoustic ray as illustrated in Fig. 8. Coordinates of a point where the straight line intersects with each of the boundaries are acquired, and distances $d_1$, $d_2$, and $d_3$ when the acoustic wave is propagated through the respective layers are calculated. Then, the propagation times $t^l$ and $t^t$ are acquired with use of an equation (8) and an equation (9), which are acquired by transforming the equation (6) and the equation (7), respectively.

$$t^l = \frac{d_1}{c_1} + \frac{d_2}{c_2^l} + \frac{d_3}{c_3} \qquad (8)$$

$$t^t = \frac{d_1}{c_1} + \frac{d_2}{c_2^t} + \frac{d_3}{c_3} \qquad (9)$$

This method can shorten the calculation time by omitting the calculation of the refraction of the acoustic ray compared to the method illustrated in Figs. 6A and 6B. This method is effective, for example, when a refraction angle is small because the acoustic velocities $c_1$, $c_2^l$, and $c_3$ of the longitudinal wave have values close to one another. Typically, the acoustic velocity $c_2^l$ in the holding cup 140 and the acoustic velocity $c_3$ in the medium are known, whereby it can be determined whether the acoustic ray may be approximated to a straight line based on the value of the acoustic velocity $c_1$ in the subject 100. Therefore, the computer 150 acquires the propagation times $t^l$ and $t^t$ by approximating the acoustic ray to a straight line when the acoustic velocity $c_1$ in the subject 100 that has been acquired in step S130 falls within a predetermined numerical range. On the other hand, the computer 150 acquires the propagation times $t^l$ and $t^t$ while taking into consideration of the refraction of the acoustic ray when the acoustic velocity $c_1$ in the subject 100 that has been acquired in step S130 falls outside the predetermined numerical range. The computer 150 may determine whether to approximate the acoustic ray according to the acoustic velocity $c_1$ for each subject 100 in this manner.

In the above-described method, the computer 150 acquires the propagation times $t^l$ and $t^t$ from the numerical calculation regarding the distance x. In addition to that, the computer 150 may analytically acquire $\theta_r$ where $x(\theta_r)$ becomes a minimum value or may numerically acquire $\theta_r$ with use of Newton's method or the like, with x expressed as a function of $\theta_r$. Alternatively, the computer 150 may utilize the fact that the propagation time t is minimized in the calculation of the acoustic ray connecting the voxel 101 and the transducer 131 to each other (Fermat's principle in geometric optics). More specifically, the computer 150 may express the propagation time t as a function regarding some geometric parameter such as a distance and an angle, and the acoustic velocity instead of the distance x, and set a minimum value of t as $t^l$ or $t^t$.

In a case where there is a plurality of positions of interest, the computer 150 may acquire the propagation times $t^l$ and $t^t$ by the above-described method for each of the positions of interest. Alternatively, the computer 150 may calculate the propagation times $t^l$ and $t^t$ with respect to a part of the positions of interest by the above-described method, and acquire the propagation times $t^l$ and $t^t$ with respect to the rest of the positions of interest by interpolation based on the calculated propagation times $t^l$ and $t^t$. Alternatively, the computer 150 may calculate the propagation times $t^l$ and $t^t$ corresponding to a position of interest by the above-described method, and also allocate the calculated propagation times $t^l$ and $t^t$ to a position of interest located around this position. In other words, the computer 150 may allocate the propagation times $t^l$ and $t^t$ corresponding to a smallest unit to another smallest unit.

<Step S150: Process for Acquiring Subject Information>

In the present process, the computer 150 calculates the initial acoustic pressure distribution in the subject 100 as the subject information with use of the two propagation times $t^l$ and $t^t$ calculated in step S140. In a case where an imaging target area includes a plurality of smallest units for the reconstruction (pixels or voxels), the computer 150 calculates the initial acoustic pressure for each of the smallest units, i.e., the initial acoustic pressure distribution in the imaging target area. Herein, this step will be described, assuming that the unit for the reconstruction is the position of interest.

In the present embodiment, an equation (10), which is acquired by transforming the equation (1), is used. In the equation (10), an index i, which indicates the number of the transducer 131, is added to each of the propagation times $t^l$ and $t^t$ as a subscript thereof ($t_i^1$ and $t_i^t$).

$$p_0(\mathbf{r}_0) = \frac{\sum_i^N \left[ b(\mathbf{r}_i, t_i^l) \cdot \Delta\Omega_i^l + b(\mathbf{r}_i, t_i^t) \cdot \Delta\Omega_i^t \right]}{\sum_i^N \left[ \Delta\Omega_i^l + \Delta\Omega_i^t \right]}$$

$$b(\mathbf{r}, t) = 2p(\mathbf{r}, t) - 2t\frac{\partial p(\mathbf{r}, t)}{\partial t} \qquad (10)$$

In other words, the computer 150 calculates the initial acoustic pressure distribution with use of both the reception signal of the acoustic wave propagated as the longitudinal wave in the holding cup 140 (hereinbelow, referred to as a longitudinal wave signal) and the reception signal of the acoustic wave propagated as the transverse wave in the holding cup 140 (hereinbelow, referred to as a transverse wave signal). More specifically, the computer 150 calculates the initial acoustic pressure distribution in the subject 100 with use of both the longitudinal wave signal and the transverse wave signal by substituting the equation (6) and the equation (7) (or the equation (8) and the equation (9)) into the equation (10). Taking into consideration the respective propagation times $t^l$ and $t^t$ of the longitudinal wave signal and the transverse wave signal allows these signals to be correctly back-projected to the voxel 101. As a result, a defocus can be prevented or reduced.

In the equation (10), different solid angle correction terms $\Delta\Omega_i$ are used for the longitudinal wave signal and the transverse wave signal. This is because the path along the acoustic ray changes between the longitudinal wave signal and the transverse wave signal, so that the solid angle is different therebetween. However, the same solid angle correction term may be employed for both the longitudinal wave signal and the transverse wave signal for the purpose of reducing a calculation load for calculating the exact solid angle. For example, a solid angle correction term acquired assuming that the acoustic ray from the position of interest to the transducer 131 is a straight line may be employed for both the longitudinal wave signal and the transverse wave signal.

Alternatively, an equation (11) may be used instead of the equation (10). In other words, the initial acoustic pressure based on the longitudinal wave and the initial acoustic pressure based on the transverse wave are calculated independently of each other, and an average thereof is calculated.

$$p_0(\mathbf{r}_0) = \frac{\dfrac{\sum_i^N b(\mathbf{r}_i, t_i^l) \cdot \Delta\Omega_i^l}{\sum_i^N \Delta\Omega_i^l} + \dfrac{\sum_i^N b(\mathbf{r}_i, t_i^t) \cdot \Delta\Omega_i^t}{\sum_i^N \Delta\Omega_i^t}}{2}$$

$$b(\mathbf{r}, t) = 2p(\mathbf{r}, t) - 2t\frac{\partial p(\mathbf{r}, t)}{\partial t} \qquad (11)$$

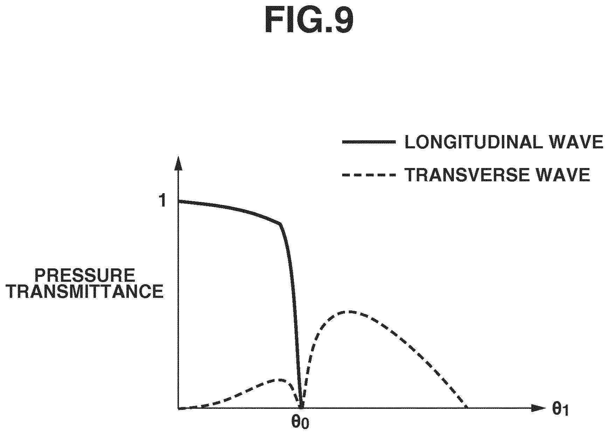

In the above-described method, the initial acoustic pressure at the position of interest is calculated with use of the longitudinal wave signal and the transverse wave signal with the same weight applied thereto, but the initial acoustic pressure at the position of interest may be calculated with different weights applied to the longitudinal wave signal and the transverse wave signal. For example, the computer 150 may determine the weights according to pressure transmittances of the longitudinal wave and the transverse wave through the holding cup 140. Fig. 9 illustrates one example of the pressure transmittances of the acoustic waves (plane waves) passing through the holding cup 140 approximated to a plane. A horizontal axis represents an incident angle $\theta_1$ of the acoustic wave incident on the holding cup 140, and a vertical axis represents the pressure transmittance of the acoustic wave. A solid line indicates the longitudinal wave, and a broken line indicates the transverse wave. In Fig. 9, $\theta_0$ indicates a critical angle of the acoustic wave (the longitudinal wave) incident on the holding cup 140. The critical angle $\theta_0$ is a total reflection angle acquired according to Snell's law. The computer 150 may use the pressure transmittance of the longitudinal wave like the example illustrated in Fig. 9 as the weight for the longitudinal wave signal and the pressure transmittance of the transverse wave like the example illustrated in Fig. 9 as the weight for the transverse wave signal. The longitudinal wave is totally reflected and only the transverse signal is propagated in an area where the incident angle $\theta_1$ is the critical angle $\theta_0$ or larger, whereby the weight for the transverse wave signal when the pressure transmittance is a predetermined value or higher may be set to 1 in this area.

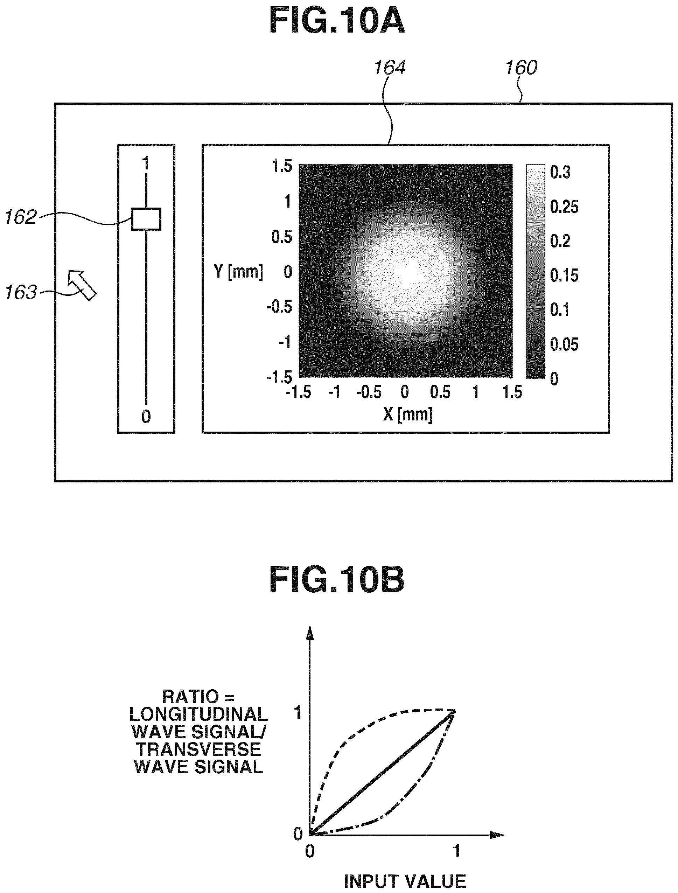

Alternatively, the user may input information regarding the weights for the longitudinal wave signal and the transverse wave signal with use of the input unit 170. For example, the input unit 170 may be configured to allow the user to input a ratio between the longitudinal wave signal and the transverse wave signal. Fig. 10A illustrates a graphical user interface (GUI) displayed on the display unit 160. The user adjusts the signal ratio by operating an icon 163 and adjusting a slide bar 162 for specifying the ratio with use of the input unit 170. Then, an image 164 of the subject information according to the specified ratio is displayed. However, the pressure transmittances of the longitudinal wave and the transverse wave with respect to the incident angle $\theta_1$ vary according to the frequency of the acoustic wave. Therefore, the weights (the ratio) applied to the longitudinal wave signal and the transverse wave signal may be changed for each frequency according to the ratio input via the input unit 170. Fig. 10B illustrates a ratio at which the acoustic wave is actually weighted with respect to the input value of the ratio. A solid line, a broken line, and a chain line each indicate a graph of the input value and the ratio for use in the processing for a different frequency component. The subject information in which a difference in the pressure transmittance for each frequency is reflected can be acquired by applying the different weights (the ratio) for each frequency with respect to the same input value in this manner. In the present embodiment, the example in which the information regarding the weights is input on the GUI has been described, but the input unit 170 may input the information by any kind of method.

The computer 150 may acquire an optical fluence distribution in the subject 100 with respect to the light 113 with which the subject 100 is irradiated, and acquire an optical absorption coefficient distribution with use of the initial acoustic pressure distribution and the optical fluence distribution.

Further or alternatively, the computer 150 may acquire a concentration distribution, such as an oxygen saturation distribution, with use of the optical absorption coefficient distribution. For example, the concentration distribution can be acquired with use of optical absorption coefficient distributions corresponding to light beams having a plurality of wavelengths.

The computer 150 outputs the subject information, such as the initial acoustic pressure distribution, the optical absorption coefficient distribution, or the concentration distribution acquired in the present process, to the display unit 160.

<Step S160: Process for Displaying Subject Information>

In the present process, the computer 150 causes the subject information in the imaging target area to be displayed on the display unit 160 with use of the subject information acquired in step S150. The initial acoustic pressure distribution, the absorption coefficient distribution, the concentration distribution (oxygen saturation distribution), or the like can be displayed as the subject information. The subject information displayed on the display unit 160 is information in which the defocus and the like are prevented or reduced, and therefore is presented as information helpful for an operator, such as a doctor, to use it for the diagnosis or the like.

In this manner, according to the method for acquiring the subject information according to the present embodiment, the subject information can be acquired with high accuracy (high image quality) while the defocus and the like are prevented or reduced therein.

A second embodiment will be described as an information acquisition apparatus capable of preventing or reducing the deterioration of the accuracy with which the subject information is acquired due to the conversion from the longitudinal wave into the transverse wave while taking a shorter calculation time compared to the first embodiment. Similar components to those of the first embodiment will be identified by the same reference numerals in principle, and descriptions thereof will be omitted.

In the present embodiment, in step S140, the finally acquired propagation time t is any one of the propagation time $t^l$ of the longitudinal wave and the propagation time $t^t$ of the transverse wave, unlike the first embodiment. In other words, it should be determined whether to use the propagation time $t^l$ of the longitudinal wave or the propagation time $t^t$ of the transverse wave as the propagation time t corresponding to the position of interest. Referring to Fig. 9, the pressure transmittance of the longitudinal wave is high for $\theta_1$ smaller than the critical angle $\theta_0$, and the pressure transmittance of the transverse wave is high for $\theta_1$ larger than $\theta_0$. Therefore, in the present embodiment, the propagation time t of the acoustic wave having a higher pressure transmittance is selectively calculated according to a magnitude relationship between $\theta_1$ and $\theta_0$.

More specifically, if the acoustic wave is incident on the holding cup 140 at the critical angle $\theta_0$ or a larger incident angle, the acoustic wave is totally reflected on the surface of the holding cup 140, so that no acoustic wave is transmitted as the longitudinal wave in the holding cup 140. On the other hand, the transverse wave has a high transmittance with the critical angle $\theta_0$ of the longitudinal wave or a larger incident angle. Therefore, the propagation time t can be calculated with use of the equations (2), (4), and (6) if the incident angle $\theta_1$ is smaller than the critical angle $\theta_0$, and with use of the equations (3), (5), and (7) if the incident angle $\theta_1$ is the critical angle $\theta_0$ or larger, with a borderline therefor set at the critical angle $\theta_0$.

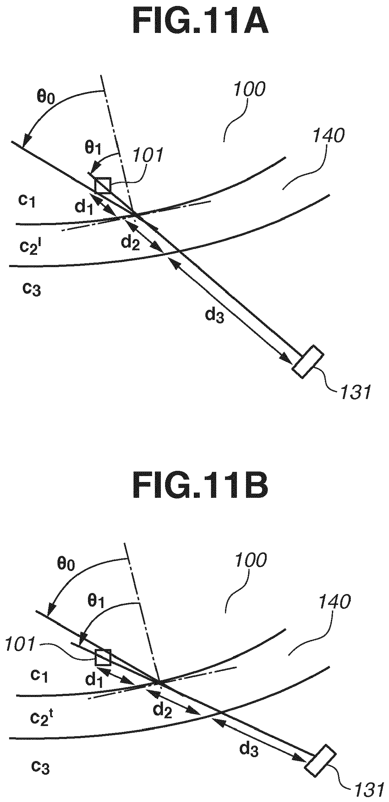

First, the computer 150 calculates the critical angle $\theta_0$ of the acoustic wave as the longitudinal wave incident from the voxel 101 onto the holding cup 140 with use of an equation (12), as illustrated in Figs. 11A and 11B.

$$\theta_0 = \sin^{-1} \frac{c_1}{c_2^l} \qquad (1\,2)$$

Next, the computer 150 calculates the incident angle $\theta_1$ formed between a straight-line acoustic ray passing through the voxel 101 and the transducer 131, and the holding cup 140.

Next, the computer 150 compares the critical angle $\theta_0$ and the incident angle $\theta_1$ with each other. Then, if $\theta_1$ is smaller than $\theta_0$, the computer 150 acquires the propagation time $t^l$ of the longitudinal wave by the calculation described in the description of the first embodiment with use of the equation (2), the equation (4), and the equation (6). On the other hand, if $\theta_1$ is $\theta_0$ or larger, the computer 150 acquires the propagation time $t^t$ of the transverse wave by the calculation described in the description of the first embodiment with use of the equation (3), the equation (5), and the equation (7). Therefore, in the case where there is the plurality of transducers 131, which propagation time is acquired varies for each of the transducers 131 with respect to the same voxel 101 depending on a positional relationship between the voxel 101 and the transducer 131.

In this manner, whether to use the propagation time $t^l$ of the longitudinal wave or the propagation time $t^t$ of the transverse wave for the calculation after that is determined based on the incident angle $\theta_1$ of the straight-line acoustic ray, which is an initial value. In this case, processing for determining a condition can be omitted during a search calculation, whereby calculation efficiency is improved. The calculation efficiency is further improved especially when the GPU is used as the processing unit.

Other than that, if the magnitude relationship between the incident angle $\theta_1$ and the critical angle $\theta_0$ varies in the course of the repeated calculation described in the description of the first embodiment, the computer 150 can employ a method that determines which is calculated, the propagation time $t^l$ of the longitudinal wave or the propagation time $t^t$ of the transverse wave, every time the calculation is repeated.

The incident angle of the refracted acoustic ray finally acquired by the above-described method often has a value close to the incident angle of the straight-line acoustic ray. Therefore, the reduction in the calculation time may be attempted by approximating the acoustic ray to a straight line without the refraction thereof calculated. If $\theta_1$ is smaller than $\theta_0$, the propagation time $t^l$ is acquired with use of the equation (8) while the acoustic velocity $c_2^l$ of the longitudinal wave is set as the acoustic velocity in the holding cup 140 as illustrated in Fig. 11A. If $\theta_1$ is $\theta_0$ or larger, the propagation time $t^t$ is acquired with use of the equation (9) while the acoustic velocity $c_2^t$ of the transverse wave is set as the acoustic velocity in the holding cup 140 as illustrated in Fig. 11B. In other words, this method eliminates the necessity of the search by the repeated calculation.

In the present embodiment, in step S150, the computer 150 calculates the initial acoustic pressure distribution with use of an equation (13) acquired by transforming the equation (1). Regarding the transducer 131 for which the propagation time $t^l$ of the longitudinal wave has been calculated in step S140, "l" is allocated to a(i). On the other hand, regarding the transducer 131 for which the propagation time $t^t$ of the transverse wave has been calculated in step S140, "t" is allocated to a(i).

$$p_0(\mathbf{r}_0) = \frac{\sum_i^N b\left(\mathbf{r}_i, t_i^{a(i)}\right) \cdot \Delta\Omega_i^{a(i)}}{\sum_i^N \Delta\Omega_i^{a(i)}}, \quad a(i) = \begin{cases} l \\ t \end{cases}$$

$$b(\mathbf{r},t) = 2p(\mathbf{r},t) - 2t\frac{\partial p(\mathbf{r},t)}{\partial t} \qquad (1\,3)$$

In other words, the computer 150 calculates the initial acoustic pressure with use of any of the longitudinal wave signal and the transverse wave signal for each transducer 131. The computer 150 calculates the initial acoustic pressure distribution in the subject 100 by substituting any one of the equation (6) and the equation (7) (or any one of the equation (8) and the equation (9)) into the equation (13) for each of the transducers 131. The same solid angle correction term may be employed for both the longitudinal wave signal and the transverse wave signal for the purpose of reducing the calculation load for calculating the exact solid angle correction term, similarly to the first embodiment.

While both the propagation times $t^l$ and $t^t$ are calculated in the first embodiment, any one of them is calculated in the present embodiment, which can shorten the calculation time. Further, this method can prevent or reduce the deterioration of the accuracy of the subject information by selecting one of the propagation times $t^l$ and $t^t$ that allows the reception signal at this time to have a higher signal-to-noise ratio (SN).

As described above, according to the method for acquiring the subject information according to the present embodiment, the calculation time necessary to acquire the initial acoustic pressure can be shortened compared to the first embodiment.

The second embodiment has been described as the embodiment in which the signal corresponding to the longitudinal wave is used when the incident angle $\theta_1$ is smaller than the critical angle $\theta_0$, and the signal corresponding to the transverse wave is used when the incident angle $\theta_1$ is equal to or larger than the critical angle $\theta_0$.

On the other hand, there is a longitudinal wave that penetrates to as deep as about one wavelength of the acoustic wave in the holding cup 140, besides the longitudinal wave as the totally reflected acoustic wave. In other words, if the holding cup 140 is as thin as or thinner than one wavelength, this longitudinal wave penetrates toward the medium filling the space between the probe 130 and the holding cup 140. The wave penetrating in the holding cup 140 in this manner is called an evanescent wave.

Typically, the evanescent wave is propagated in a normal direction with respect to the surface of the holding cup 140. In this case, an amplitude $A_1$ of the evanescent wave when this wave penetrates through the holding cup 140 is expressed by an equation (14).

$$A_l = A_0 \exp\left(-\sqrt{k_x^2 + k_y^2 - \left(\frac{\omega}{c_2^l}\right)^2} \cdot L\right) \qquad (14)$$

In this equation (14), $A_0$, $k_x$, $k_y$, and $\omega$ represent the amplitude of the acoustic wave on the surface of the holding cup 140 when this wave is incident on the holding cup 140, a wavenumber in the x direction in the subject 100, a wavenumber in the y direction in the subject 100, and an angular frequency of the acoustic wave, respectively. Further, in this equation (14), $c_2^l$ and L represent the acoustic velocity of the longitudinal wave in the holding cup 140 and the thickness of the holding cup 140, respectively. According to the equation (14), it is understood that the amplitude $A_1$ of the penetrating evanescent wave reduces as the thickness L of the holding cup 140 increases. The wave numbers $k_x$ and $k_y$ can be acquired from the incident angle $\theta_l$.

If an evanescent wave of the transverse wave is generated, the evanescent wave may be acquired by replacing the acoustic velocity $c_2^l$ of the longitudinal wave in the equation (14) with the acoustic velocity $c_2^t$ of the transverse wave. Further, if the evanescent wave is propagated in a different direction from a direction in which the acoustic wave is being attenuated, the transmittance may be estimated by estimating an attenuation amount corresponding to this propagation path.

The thickness L of the holding cup 140 desirable for implementation of a third embodiment will be described. The thickness L of the holding cup 140 desirable for implementation of the present embodiment is determined according to the frequency band of the acoustic wave desired to be used to acquire the subject information, and the acoustic velocity $c_2^l$ of the longitudinal wave in the holding cup 140. Now, suppose an example in which the used holding cup 140 is a holding cup having a similar acoustic velocity of the longitudinal wave to the typical living body (1500 m/s). In this case, it is desirable to implement the present embodiment when the thickness L of the holding cup 140 is 1 mm or thinner if the subject information is acquired with use of the acoustic wave having a frequency band up to about 1.5 MHz. Further, it is desirable to implement the present embodiment when the thickness L of the holding cup 140 is 0.15 mm or thinner if the subject information is acquired with use of the acoustic wave having a frequency band up to about 10 MHz. Further, it is desirable to implement the present embodiment when the thickness L of the holding cup 140 is 0.03 mm or thinner if the subject information is acquired with use of the acoustic wave having a frequency band up to about 50 MHz. Typically, using the high frequency component of the acoustic wave allows the information to be acquired at a high resolution.

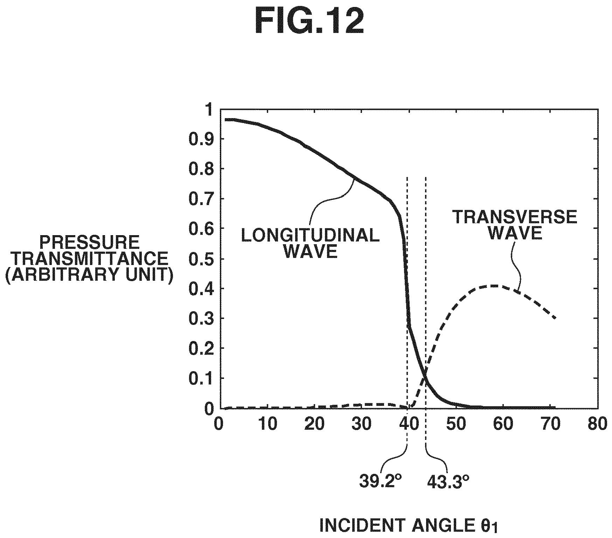

Fig. 12 illustrates a result of a simulation of the pressure transmittances of the longitudinal wave and the transverse wave with respect to the incident angle $\theta_1$ at which the acoustic wave is incident on the holding cup 140. Fig. 12 illustrates the pressure transmittances also including the amplitude $A_1$ of the evanescent wave penetrating through the holding cup 140 indicated in the equation (14). This simulation is conducted assuming that the respective acoustic velocities are $c_1 = 1480$ m/s, $c_2^l = 2340$ m/s, $c_2^t = 912$ m/s, and $c_3 = 1500$ m/s. Further, this simulation is conducted assuming that the thickness L of the holding cup 140 is L = 500 $\mu$m. The calculation is made assuming that the frequency contained in the acoustic wave is 0 to 10 MHz at this time. The critical angle $\theta_0$ acquired with use of the equation (12) according to Snell's law from these parameters is 39.2 degrees. However, according to Fig. 12, it is understood that the longitudinal wave penetrates as the evanescent wave even with the critical angle $\theta_0$ or a larger incident angle. According to Fig. 12, it is understood that the longitudinal wave maintains a higher pressure transmittance than the transverse wave until the incident angle $\theta_1$ reaches an incident angle of 43.3 degrees, which is larger than the critical angle $\theta_0$ of 39.2 degrees.

In a case where the holding cup 140 is thick (thicker than one wavelength of the acoustic wave), the longitudinal wave is totally reflected and the transmittance of the transverse wave increases at an incident angle where the longitudinal wave loses the transmittance. Therefore, the image quality is unlikely to reduce, even when the calculation is made, assuming that a magnitude relationship is reversed between the transmittances of the longitudinal wave and the transverse wave at the critical angle $\theta_0$. However, in the case where the thickness L of the holding cup 140 is thin (as thin as or thinner than one wavelength of the acoustic wave), the magnitude relationship is reversed between the transmittance of the longitudinal wave and the transmittance of the transverse wave at the critical angle $\theta_0$ or a larger incident angle, as illustrated in Fig. 12. Therefore, a term "hypothetical critical angle" is used to refer to an angle larger than the critical angle $\theta_0$ and at which a degree of influence of the transmittance of the longitudinal wave sufficiently reduces compared to a degree of influence of the transmittance of the transverse wave. For example, the incident angle at which the magnitude relationship is reversed between the transmittance of the longitudinal wave and the transmittance of the transverse wave with the evanescent wave taken into consideration may be set as the "hypothetical critical angle".

<Method for Setting Hypothetical Critical Angle>

An example of a method for setting the hypothetical critical angle will be described. The computer 150 as an angle setting unit can calculate the transmittance of the longitudinal wave and the transmittance of the transverse wave in the holding cup 140, and set the hypothetical critical angle based on these transmittances.

First, the computer 150 acquires parameters necessary to calculate the transmittances. For example, the computer 150 acquires the information indicating the acoustic velocity $c_1$ of the longitudinal wave in the subject 100, the information indicating the acoustic velocity $c_2^l$ of the longitudinal wave and the acoustic velocity $c_2^t$ of the transverse wave in the holding cup 140, and the information regarding the thickness L of the holding cup 140.

The acoustic velocity information may be acquired by the method described in step S130. Alternatively, the user may input these parameters with use of the input unit 170, and the computer 150 may acquire these parameters by receiving the information output from the input unit 170. Alternatively, the computer 150 may acquire the parameters by reading out the parameters stored in the storage unit in advance. Alternatively, the readout unit 143 may read out the tag 142 in which the parameters regarding the holding cup 140 are recorded, and the computer 150 may acquire the parameters regarding the holding cup 140 by receiving the information output from the readout unit 143. The computer 150 may also acquire a parameter necessary to calculate the transmittances, besides the above-described parameters.

Next, the computer 150 calculates the transmittances of the longitudinal wave and the transverse wave for each angle at which the acoustic wave is incident on the holding cup 140 with use of the information regarding the above-described parameters. For example, the computer 150 calculates the transmittances of the longitudinal wave and the transverse wave when the incident angle $\theta_1$ changes from 0 degrees to 70 degrees at 10 degree intervals. The transmittance at the incident angle that is not directly calculated may be interpolated from the transmittances at the incident angles previous and subsequent thereto. The computer 150 can find out the hypothetical critical angle with a small calculation amount by selectively interpolating an incident angle between two incident angles between which the magnitude relationship is reversed between the transmittances of the longitudinal wave and the transverse wave, among the discretely calculated transmittances. The result of the transmittances like the example illustrated in Fig. 12 can be acquired from these calculations. Here, the pressure transmittance is desirable as the transmittance for use in the calculation of the hypothetical critical angle. This is because the probe 130, which is the reception unit, yields an output proportional to the pressure of the acoustic wave.

Next, the computer 150 calculates the incident angle at which the magnitude relationship is reversed between the transmittances of the longitudinal wave and the transverse wave from the result of the calculation of the transmittances illustrated in Fig. 12, and sets this incident angle as the hypothetical critical angle. In Fig. 12, 43.3 degrees is acquired as the hypothetical critical angle.

The hypothetical critical angle may be not only the incident angle at which the magnitude relationship is reversed between the transmittances of the longitudinal wave and the transverse wave but also an angle around this incident angle. For example, the computer 150 may set the hypothetical critical angle within a range of $\pm$ 1 degrees from the incident angle at which the magnitude relationship is reversed between the transmittances of the longitudinal wave and the transverse wave. Alternatively, the computer 150 may set an incident angle at which the transmittance of the longitudinal wave reduces to a threshold value or lower (e.g., 10% or lower) as the hypothetical critical angle. Alternatively, the computer 150 may set an incident angle at which the transmittance of the transverse wave increases to a threshold value or higher (e.g., 10% or higher) as the hypothetical critical angle.

Alternatively, the hypothetical critical angle may be set in the following manner. The computer 150 acquires an image acquired with use of the signal corresponding to the longitudinal wave when the incident angle $\theta_1$ is the critical angle $\theta_0$ or larger, and acquires image quality information of this image. Then, the computer 150 evaluates the image quality information, and sets an incident angle when the image quality information falls within a predetermined numerical range (e.g., 90% of the specifications of the apparatus) as the hypothetical critical angle. If the image quality is larger than a threshold value, the computer 150 sets a larger hypothetical critical angle again, and evaluates the image quality information of the image acquired with use of the hypothetical critical angle set again. The computer 150 repeats this procedure. In the present example, the setting of the hypothetical critical angle has been described based on the processing that is updating the hypothetical critical angle to a larger angle, but the hypothetical critical angle may be set by processing that is updating the hypothetical critical angle to a smaller angle. For example, the image quality information refers to information indicating at least one of the contrast and the resolution.

Alternatively, the user may input the hypothetical critical angle based on which the use of the longitudinal wave or the transverse wave is switched, with use of the input unit 170. Further, the input unit 170 may be configured to allow the user to input the hypothetical critical angle while the subject information is displayed on the display unit 160. Then, the computer 150 may generate an image based on the input hypothetical critical angle, and cause this image to be displayed on the display unit 160. With this display, the user can search for an appropriate hypothetical critical angle while confirming a change in the image of the subject information according to the input hypothetical critical angle.

The computer 150 switches a mode to a mode of acquiring the subject information with use of only the signal corresponding to the transverse wave when the incident angle $\theta_1$ exceeds the hypothetical critical angle. The computer 150 can determine the magnitude relationship between the incident angle $\theta_1$ and each of the critical angle $\theta_0$ and the hypothetical critical angle, and determine the signal for use in the acquisition of the subject information according to a result of this determination.

<Method for Acquiring Propagation Time Information>

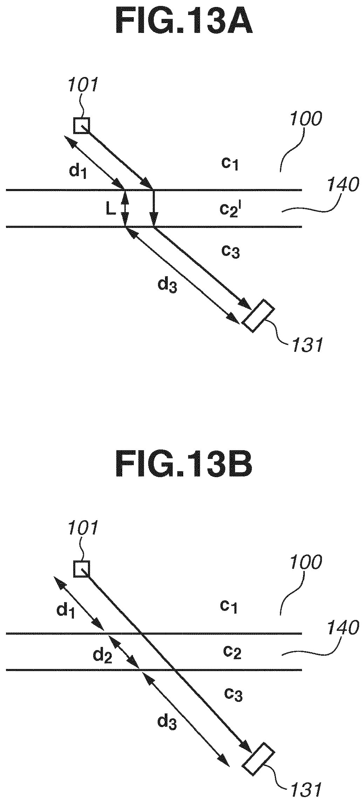

In the present embodiment, if the acoustic wave is incident at a smaller incident angle than the hypothetical critical angle, the computer 150 calculates the propagation time t assuming that the acoustic wave in the holding cup 140 is the longitudinal wave. On the other hand, if the acoustic wave is incident at an incident angle equal to or larger than the hypothetical critical angle, the computer 150 calculates the propagation time t assuming that the acoustic wave in the holding cup 140 is the transverse wave. The computer 150 may calculate the propagation time t by tracing the acoustic ray propagated as the evanescent wave in the holding cup 140 as illustrated in Fig. 13A, when the incident angle $\theta_1$ is larger than the critical angle $\theta_0$.

The computer 150 determines whether the incident angle $\theta_1$ at which the acoustic wave is incident on the holding cup 140 is larger or smaller than the critical angle $\theta_0$ and whether the incident angle $\theta_1$ is larger or smaller than the hypothetical critical angle, and changes the method for acquiring the propagation time t according to results of these determinations.

If the incident angle $\theta_1$ is smaller than the critical angle $\theta_0$, the computer 150 acquires the propagation time t according to the equation (6) or the equation (8) using the acoustic velocity $c_2^l$ of the longitudinal wave in the holding cup 140 as described in the first embodiment.

If the incident angle $\theta_1$ is equal to or larger than the critical angle $\theta_0$ and smaller than the hypothetical critical angle, the computer 150 acquires the propagation time t assuming that the acoustic velocity in the holding cup 140 is the acoustic velocity $c_2^l$ of the longitudinal wave. For example, the computer 150 as a first determination unit traces a propagation path specific to the evanescent wave along the acoustic ray as illustrated in Fig. 13A to acquire the propagation time t. In this case, the computer 150 can acquire the propagation time t according to an equation (15).

$$t^l = \frac{d_1}{c_1} + \frac{L}{c_2^l} + \frac{d_3}{c_3} \qquad (15)$$

The computer 150 can determine a first signal of interest corresponding to the propagation time t expressed by the equation (15) from among the reception signals acquired in step S120, and acquire the subject information at the voxel 101 with use of this signal.

Further, if the holding cup 140 is thin enough to allow the evanescent wave to penetrate therethrough (if the holding cup 140 is as thin as or thinner than one wavelength of the wavelength of the acoustic wave), the propagation path of the evanescent wave little affects the propagation time t. Therefore, the computer 150 may acquire the propagation time t based on the straight-line approximation as illustrated in Fig. 13B. In this case, the computer 150 may acquire the propagation time t according to the equation (8).

If the incident angle $\theta_1$ is larger than the hypothetical critical angle, the computer 150 as a second determination unit acquires the propagation time t according to the equation (7) or (9) using the acoustic velocity $c_2^t$ of the transverse wave in the holding cup 140 as described in the description of the first embodiment. The computer 150 can determine a second signal of interest corresponding to the propagation time t expressed by the equation (7) or (9) from among the reception signals acquired in step S120, and acquire the subject information at the voxel 101 with use of this signal.

The computer 150 can acquire the subject information with use of the longitudinal wave signal regarding a reconstruction position where the incident angle $\theta_1$ is smaller than the critical angle $\theta_0$ (first reconstruction position). The computer 150 may use the transverse wave signal or may refrain from using the transverse wave signal when acquiring the subject information at the first reconstruction position. Further, the computer 150 can acquire the subject information with use of the transverse wave signal without use of the longitudinal wave signal regarding a reconstruction position where the incident angle $\theta_1$ is larger than the hypothetical critical angle (second reconstruction position). Further, the computer 150 can acquire the subject information with use of both the longitudinal wave signal and the transverse wave signal regarding a reconstruction position where the incident angle $\theta_1$ is larger than the critical angle $\theta_0$ and is smaller than the hypothetical critical angle (third reconstruction position).

This method allows the subject information to be acquired with higher accuracy than calculating the propagation time t while switching the acoustic wave between the longitudinal wave and the transverse wave based on the critical angle $\theta_0$.

[Example]

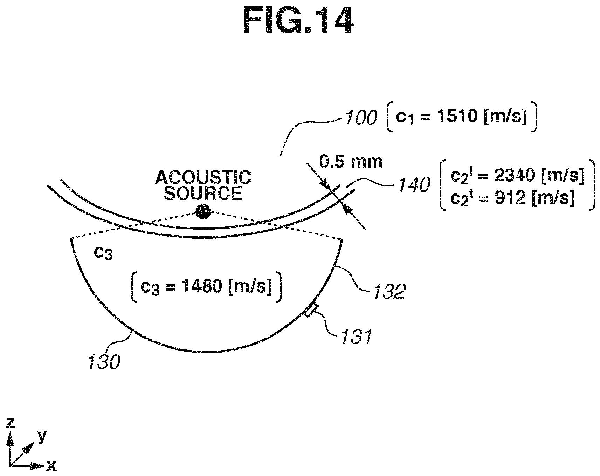

In the following description, a result of simulating the method for acquiring the subject information according to the third embodiment will be described. Fig. 14 illustrates a calculation model for the simulation. The acoustic source is located at the center of the curvature of the spherical probe 130, is $\varphi$ 5 mm in diameter, and generates an initial acoustic pressure of 1 Pa. The acoustic velocity $c_1$ in the subject 100 where the acoustic source is located is $c_1$ = 1510 m/s. The holding cup 140 is 0.5 mm in thickness, and the acoustic velocity $c_2^l$ of the longitudinal wave and the acoustic velocity $c_2^t$ of the transverse wave in the holding cup 140 are $c_2^l$ = 2340 m/s and $c_2^t$ = 912 m/s, respectively. The space between the probe 130 and the holding cup 140 is filled with the medium in which the acoustic velocity $c_3$ is $c_3$ = 1480 m/s. The probe 130 includes 512 transducers 131, which are evenly and spirally arranged on the spherical surface of the probe 130.

In such a calculation model, the reception signal of each of the transducers 131 is generated based on an analytical solution in which a part of the longitudinal wave is converted into the transverse wave in the holding cup 140.

Next, the image of the acoustic source (the initial acoustic pressure thereof) is reconstructed with use of the generated reception signal by the calculation method according to the present embodiment. The reconstructed area is a cube volume of 3 × 3 × 3 mm centered at the position where the acoustic source is placed. A voxel size for the reconstruction is 0.1 mm.

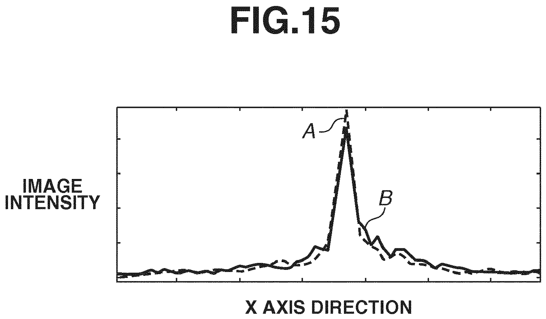

Fig. 15 illustrates image intensity profiles of pieces of three-dimensional volume data acquired by performing different image reconstructing methods with respect to the calculation model illustrated in Fig. 14. Fig. 15 illustrates an image intensity profile generated by carrying out maximum intensity projection (MIP) on each of the pieces of three-dimensional volume data in the z direction, and constructing an image intensity profile in the x axis direction at the position where the center of the absorber is located. A solid line B illustrated in Fig. 15 indicates an intensity profile of an image generated while the use of the longitudinal wave signal and the use of the transverse wave signal are switched based on the critical angle $\theta_0$ according to Snell's law. A broken line A illustrated in Fig. 15 indicates an intensity profile of an image generated while the use of the longitudinal wave signal and the use of the transverse wave signal are switched based on the hypothetical critical angle, which is larger than the critical angle $\theta_0$, according to Snell's law, like the third embodiment.

According to Fig. 15, it is understood that the intensity profile indicated by the broken line A exhibits a higher peak intensity compared to the intensity profile indicated by the solid line B. Further, according to Fig. 15, it is understood that the intensity indicated by the broken line A is less defocused and includes fewer peripheral artifacts compared to the intensity profile indicated by the solid line B. From these observations, it is understood that the subject information may be acquired with higher accuracy by switching the use of the longitudinal wave signal and the use of the transverse wave signal based on the hypothetical critical angle, which is larger than the critical angle $\theta_0$, than by switching the use of the longitudinal wave signal and the use of the transverse wave signal based on the critical angle $\theta_0$.

A fourth embodiment will be described as an information acquisition apparatus capable of acquiring the subject information with high accuracy (high image quality) while shortening the calculation time by preparing a table storing the propagation time t in advance. Similar components to those in the first to third embodiments will be identified by the same reference numerals, and descriptions thereof will be omitted.

In the present embodiment, in step S140, the computer 150 acquires the propagation time t by referring to a propagation time table stored in the storage unit in the computer 150.

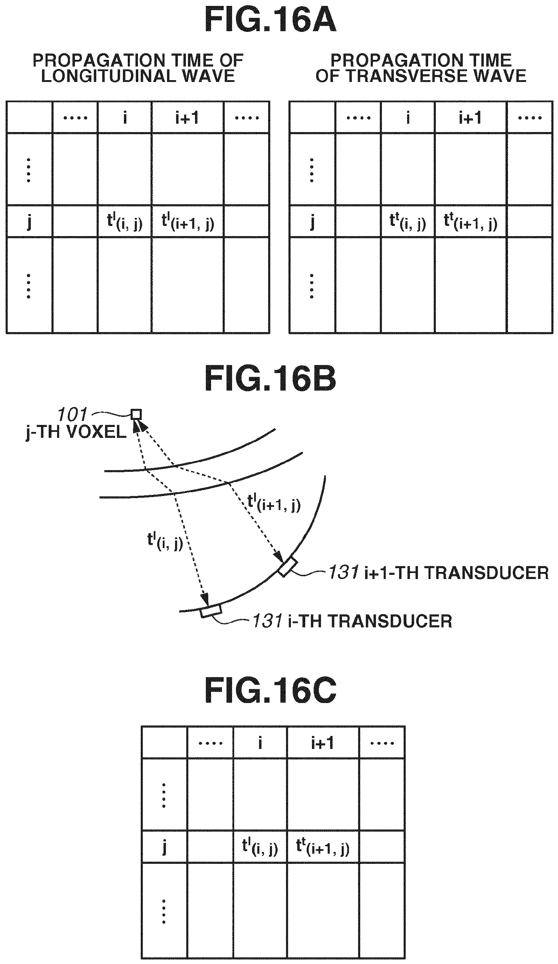

Fig. 16A illustrates an example of the propagation time table. In the following description, the present embodiment will be described, assuming that there are a plurality of voxels 101 and a plurality of transducers 131. The propagation time table includes two tables, i.e., a table storing the propagation time $t^l$ of the longitudinal wave, and a table storing the propagation time $t^t$ of the transverse wave. A row and a column indicate a number of each of the voxels 101 and a number of each of the transducers 131, respectively, and the propagation time t is stored in a corresponding field. For example, $t^l(i, j)$ and $t^l(i + 1, j)$ illustrated in Fig. 16A correspond to propagation times $t^l$ of longitudinal waves in a layout illustrated in Fig. 16B. The propagation time table is calculated in advance by the method described in the description of the first embodiment, and is stored in a propagation time storage unit 190. The stored value may be the propagation time of the refracted acoustic ray, or may be the propagation time of the acoustic ray approximated to a straight line.

The computer 150 acquires the respective propagation times $t^l$ and $t^t$ of the longitudinal wave and the transverse wave corresponding to each of the transducers 131 from the propagation time table according to the number of the voxel 101 from which the computer 150 attempts to acquire the subject information. The use of the propagation time t calculated in advance in this manner can shorten the calculation time necessary to acquire the propagation time t compared to the first embodiment.

The propagation time table may store the propagation time t with respect to only a part of the plurality of voxels 101 and/or only a part of the plurality of transducers 131. In this case, the computer 150 may acquire the not-stored propagation time t by interpolation with use of the stored propagation time t. In this case, a memory capacity occupied by the propagation time table can be reduced compared to storing all the propagation times t.

Typically, the propagation time table is calculated in advance regarding a representative acoustic velocity in the subject 100 (e.g., an average acoustic velocity in the living body), and is stored in the propagation time storage unit 190. However, the propagation time t varies according to the acoustic velocity ($c_1$, $c_2^l$, $c_2^t$, and $c_3$), whereby the propagation time table may be prepared and stored in the propagation time storage unit 190 for each acoustic velocity. The table for each acoustic velocity may be prepared with respect to all of $c_1$, $c_2^l$, $c_2^t$, and $c_3$, or the reduction in the occupied memory capacity may be attempted by preparing the table for each acoustic velocity with respect to only the acoustic velocity $c_1$, with which the propagation time t more largely varies according to individual variability of the subject 100 than the other acoustic velocities. The computer 150 may acquire the propagation time t by referring to the table corresponding to $c_1$ acquired in step S130. In this case, the computer 150 can simply acquire the propagation time t without requiring an increase in the size of the apparatus, by employing the method for acquiring the acoustic velocity $c_1$ in the subject 100 with use of the acoustic wave generated in the subject 100.

Further or alternatively, a table indicating a relationship of the propagation time t to the temperature of the holding cup 140 may be stored in the storage unit in advance. Then, in the present process, the temperature measurement unit may measure the temperature of the holding cup 140, and the computer 150 may acquire the propagation time t corresponding to the measured temperature according to the equation or the table indicating the relationship.

Alternatively, the computer 150 may acquire the propagation time t corresponding to the holding cup 140 mounted on the mounting unit 141. For example, the readout unit 143 may read out the propagation time table in the holding cup 140 that is registered on the tag 142 of the holding cup 140, and transfer the read propagation time table to the computer 150. The computer 150 may acquire the propagation time table read out by the readout unit 143. Alternatively, the user may input the ID for identifying the holding cup 140 that is allocated to the holding cup 140 via the input unit 170, and the computer 150 may acquire the propagation time t by reading out the propagation time table corresponding to the input ID for identifying the holding cup 140 from the storage unit.

The computer 150 may acquire the propagation time t by reading out the propagation time table corresponding to the acoustic velocity $c_1$ in the subject 100 that has been acquired in step S130 and the holding cup 140 mounted on the mounting unit 141 from the storage unit, and referring to the read table.

Further, the reduction in the occupied memory capacity may be attempted by preparing a table or tables corresponding to several acoustic velocities and interpolating the propagation time t regarding an acoustic velocity between them by the computer 150 to acquire the propagation time t.

Further, the propagation time table can also be prepared by the method described in the description of the second embodiment or the third embodiment. As illustrated in Fig. 16C, any one of the propagation time $t^l$ of the longitudinal wave and the propagation time $t^t$ of the transverse wave is stored in each field, so that only a single table is prepared. According to this method, the memory capacity occupied by the propagation time table can be reduced compared to storing both the propagation time $t^l$ of the longitudinal wave and the propagation time $t^t$ of the transverse wave.

In this manner, according to the method for acquiring the subject information according to the present embodiment, the subject information can be acquired with high accuracy (a high image quality) while the calculation time necessary to acquire the propagation time t is shortened.

A fifth embodiment will be described as an apparatus that irradiates the subject 100 with a spherical acoustic wave and acquires the subject information with use of a signal acquired by receiving the acoustic wave reflected and propagated inside the subject 100. The apparatus according to the present embodiment can image a reflector of the acoustic wave. In other words, the apparatus can acquire information indicating an acoustic reflection distribution regarding a difference in acoustic impedance as the subject information. With the apparatus according to the present embodiment, the acoustic velocity in the subject 100, Doppler information, elasticity information, or the like can be acquired as the subject information besides the acoustic reflection distribution.

Similar components to those of the first to fourth embodiments will be identified by the same reference numerals, and descriptions thereof will be omitted.

<Configuration of Information Acquisition Apparatus>

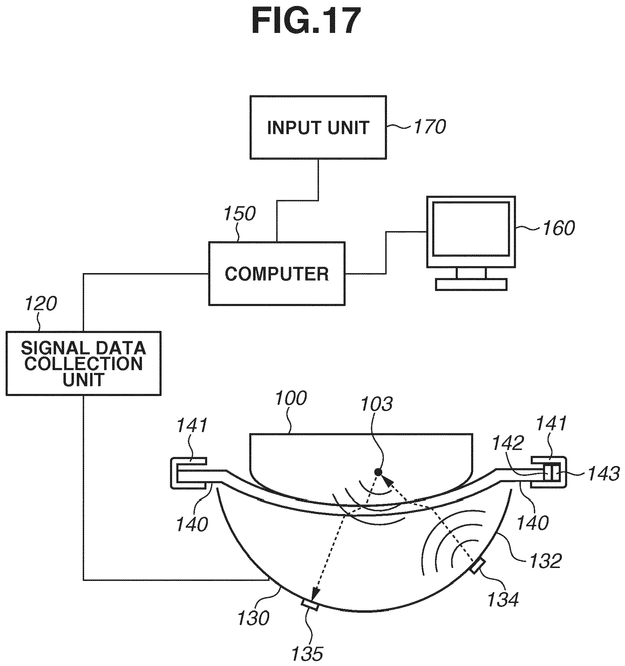

Fig. 17 is a schematic diagram of an information acquisition apparatus according to the present embodiment. Now, each of components of the present apparatus will be described below. The present apparatus includes the probe 130 including transducers 134 and 135, the holding cup 140, the signal data collection unit 120, the computer 150, the display unit 160, and the input unit 170. The measurement target is the subject 100.

A piezoelectric ceramic material represented by PZT, a polymer piezoelectric film material represented by PVDF, or the like can be used as a member forming each of the transducers 134 and 135. Alternatively, an element other than the piezoelectric element may be used. For example, an electrostatic capacitance type transducer (CMUT) or a transducer using a Fabry-Perot interferometer can be used. Any transducer may be employed as long as this transducer can transmit the acoustic wave by receiving an electric signal or output an electric signal by receiving the acoustic wave. Further, the signal acquired by the transducer 134 or 135 is a time-resolved signal. In other words, an amplitude of the signal acquired by the reception element indicates a value based on the acoustic pressure (e.g., a value proportional to the acoustic pressure) received by the transducer 134 or 135 at each time. The transducer 134 or 135 may transmit or receive an acoustic wave having a frequency from several kHz to several hundred MHz, which are commonly used in an ultrasonographic apparatus. In the present embodiment, the information acquisition apparatus will be described based on an example in which the transducer 134 transmits the acoustic wave, and the transducer 135 receives the acoustic wave (an echo). In other words, in the present embodiment, the probe 130 functions as both the reception unit and an ultrasonic irradiation unit (a transmission unit). The probe 130 may include another transducer besides the transducer 134 and the transducer 135. This configuration allows the information acquisition apparatus to acquire the reflected and propagated acoustic wave at a plurality of positions by irradiating the subject 100 with the acoustic wave once, thereby contributing to an increase in an information amount usable to form the image and thus improvement of the image quality. Further, the probe 130 may irradiate the subject 100 with the acoustic wave a plurality of times while changing the transducer by which the subject 100 is irradiated with the acoustic wave.

The control unit of the computer 150 drives the transducer 134 or the transducer 135 by an electric signal to cause it to generate the acoustic wave. The computer 150 may include an amplification circuit for amplifying this electric signal. The compute 150 may transmit a pulsed electric signal to the transducer 134, and cause the transducer 134 to transmit a pulsed acoustic wave.

<Method for Acquiring Subject Information>

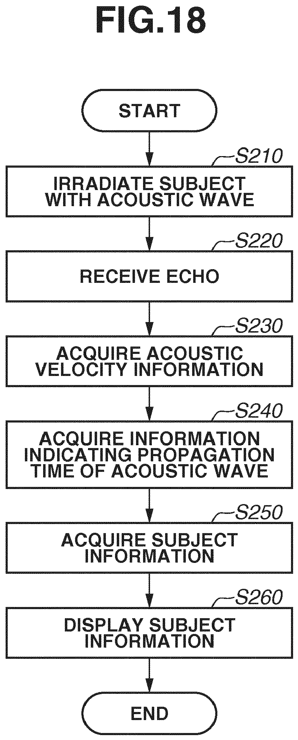

Next, each of processes in a method for acquiring the subject information according to the present embodiment will be described with reference to Fig. 18. The computer 150 controls the operation of each of the units of the information acquisition apparatus, by which each of the processes is performed.

<Step S210: Process for Irradiating Subject 100 with Acoustic Wave>

In the present process, the transducer 134 transmits the pulsed acoustic wave, and irradiates the subject 100 held in the holding cup 140 therewith. At this time, the transducer 134 transmits the spherical acoustic wave. The transmitted acoustic wave is propagated in the subject 100 while being reflected or scattered by a reflector 103 or the like inside the subject 100. Then, the transmitted acoustic wave is reflected by the reflector 103 in the subject 100, and is propagated to the probe 130 while taking the form of the echo. The reflector 103 existing inside the subject 100 is a substance having acoustic impedance relatively largely different from a surrounding area inside the subject 100. For example, in the case where the living body is handled as the measurement target, calcification caused by a malignant tumor or the like serves as the reflector 103.

<Step S220: Process for Receiving Echo>

In the present process, the probe 130 receives the acoustic wave (the echo) and outputs the chronological electric signal. The probe 130 receives the acoustic wave propagated in the subject 100 after the subject 100 has been irradiated with the acoustic wave in step S210, and outputs the reception signal from the transducer 135. The signal output from the probe 130 is transferred to the computer 150. Steps S210 and S220 may be performed a plurality of times while the transducer that irradiates the subject 100 with the acoustic wave is changed.

<Step S230: Process for Acquiring Acoustic Velocity Information>

The computer 150 acquires the acoustic velocity information in the subject 100, the holding cup 140, the medium, and the like by a similar method to step S130.

<Step S240: Process for Acquiring Propagation Time of Acoustic Wave>

In the present process, the computer 150 acquires a propagation time of the acoustic wave from the transmission of the acoustic wave from the probe 130 to the reception of the echo generated by the reflection of the acoustic wave on the reflector 103.

Generally, an equation (16) can be used as a method for calculating the acoustic reflection distribution.

$$I(\mathbf{r}_0) = \sum_i^N p\left(\mathbf{r}_i, t = \frac{|\mathbf{r}_k - \mathbf{r}_0| + |\mathbf{r}_i - \mathbf{r}_0|}{c}\right) \qquad (1\,6)$$

In this equation (16), $r_0$ represents the positional vector indicating the position to be reconstructed, $I(r_0, t)$ represents a value related to the acoustic reflection at the position to be reconstructed, $r_i$ represents a positional vector of the transducer 135, $r_k$ represents a positional vector of the transducer 134 that transmits the acoustic wave, and c represents the acoustic velocity in the subject 100. Further, N represents the number of transducers 135 for use in the reconstruction. The equation (16) indicates that reception signals $p(r_i, t)$ are subjected to processing such as a correction of attenuation of the spherical wave, and are subjected to phasing and summing while a propagation time of the transmission and a propagation time of the reception are taken into consideration (back-projection). In the equation (16), t represents a total of a time taken for the acoustic wave to be propagated from the transducer 134 to the position of interest, and a time taken for the acoustic wave to be propagated from the position of interest to the transducer 135. Calculation processing may be performed on $p(r_i, t)$. Examples of the calculation processing include frequency filtering (low-pass filtering, high-pass filtering, band-pass filtering, or the like), deconvolution, envelop detection, wavelet filtering, and time gain control (TGC). Further, apodization may be carried out in the calculation of $I(r_0)$.

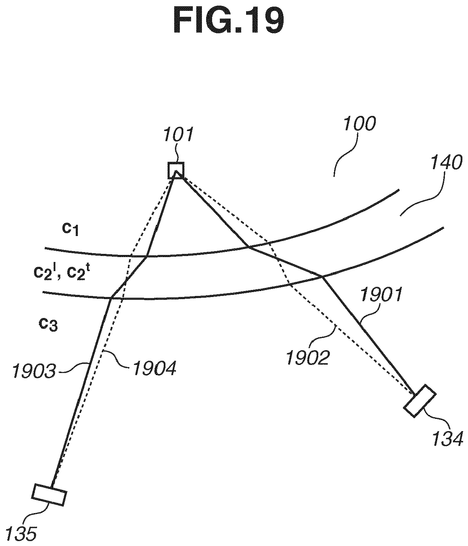

In the present process, the computer 150 calculates propagation times $t^{ll}$, $t^{lt}$, $t^{tl}$, and $t^{tt}$. A first subscript added to the propagation time t indicates whether the acoustic wave is propagated as the longitudinal wave or is propagated as the transverse wave in the holding cup 140 when traveling from the transducer 134 to the subject 100. A second subscript added to the propagation time t indicates whether the acoustic wave is propagated as the longitudinal wave or is propagated as the transverse wave in the holding cup 140 when traveling from the subject 100 to the transducer 135. A subscript 1 indicates the longitudinal wave, and a subscript t indicates the transverse wave. For example, $t^{lt}$ indicates the propagation time along an acoustic ray propagated as the longitudinal wave in the holding cup 140 when the acoustic wave travels from the transducer 134 to the subject 100, and propagated as the transverse wave in the holding cup 140 when the acoustic wave travels from the subject 100 to the transducer 135. A method for calculating them will be described with reference to Fig. 19.

In Fig. 19, an acoustic ray 1901 is an acoustic ray propagated as the longitudinal wave in the holding cup 140 when the acoustic wave travels from the transducer 134 to the subject 100. An acoustic ray 1902 is an acoustic ray propagated as the transverse wave in the holding cup 140 when the acoustic wave travels from the transducer 134 to the subject 100. An acoustic ray 1903 is an acoustic ray propagated as the longitudinal wave in the holding cup 140 when the acoustic wave travels from the subject 100 to the transducer 135. An acoustic ray 1904 is an acoustic ray propagated as the transverse wave in the holding cup 140 when the acoustic wave travels from the subject 100 to the transducer 135. In other words, in the present embodiment, the computer 150 calculates the propagation time of each of the acoustic rays in consideration of the four patterns of acoustic rays.

Each of the propagation times $t^{ll}$, $t^{lt}$, $t^{tl}$, and $t^{tt}$ is a total of the propagation time of any of the acoustic ray 1901 and the acoustic ray 1902, and the propagation time of any of the acoustic ray 1903 and the acoustic ray 1904.

The propagation times of the acoustic ray 1901 and the acoustic ray 1903 can be acquired by performing the calculation method described in the description of the first embodiment, assuming that the acoustic velocity in the holding cup 140 is the acoustic velocity $c_2^l$ of the longitudinal wave. The propagation times of the acoustic ray 1902 and the acoustic ray 1904 can be acquired by performing the calculation method described in the description of the first embodiment, assuming that the acoustic velocity in the holding cup 140 is the acoustic velocity $c_2^t$ of the transverse wave. In the case where the refracted acoustic ray is acquired by the calculation method according to the first embodiment, the acoustic ray 1901 and the acoustic ray 1902 are calculated as if they are propagated in an opposite direction from the direction in which they are actually propagated, but the finally acquired acoustic wave is not affected by the propagation direction, so that no problem arises therefrom.

Alternatively, the respective propagation times may be acquired by referring to the propagation time table as described in the description of the fourth embodiment.

<Step S250: Process for Acquiring Subject Information>

In the present process, the computer 150 acquires information indicating the acoustic reflection distribution in the subject 100 as the subject information with use of the four propagation times $t^{ll}$, $t^{lt}$, $t^{tl}$, and $t^{tt}$ calculated in step S240. In the case where the imaging target area includes a plurality of smallest units for the reconstruction (pixels or voxels), the computer 150 calculates a value regarding the acoustic reflection for each of the smallest units, i.e., the acoustic reflection distribution in the imaging target area.

In the present embodiment, an equation (17), which is acquired by transforming the equation (16), is used.

$$I(\mathbf{r}_0) = \sum_i^N \left[ p(\mathbf{r}_i, t^{ll}) + p(\mathbf{r}_i, t^{lt}) + p(\mathbf{r}_i, t^{tl}) + p(\mathbf{r}_i, t^{tt}) \right] \qquad (1\ 7)$$

In other words, taking into consideration all of the propagation times $t^{ll}$, $t^{lt}$, $t^{tl}$, and $t^{tt}$ brought about from the combinations of the respective longitudinal wave signals and the respective transverse wave signals of the transmission and the reception allows these signals to be correctly back-projected to the voxel 101. As a result, the defocus can be prevented or reduced. The weight allocated to each of the signals may be changed as described in the description of the first embodiment. Further, not all of the four terms in the equation (17) have to be used. For example, one of the longitudinal wave signal and the transverse wave signal may be selectively back-projected with respect to each of the transmission and the reception, similarly to the second embodiment. In this case, the defocus can be prevented or reduced while the time taken to calculate the propagation time t in step S240 and the time taken to calculate the back-projection in step S250 are shortened.

<Step S260: Process for Displaying Subject Information>

In the present process, the information indicating the acoustic reflection distribution that has been acquired in step S250 is displayed on the display unit 160 as the subject information of the imaging target area. The subject information displayed on the display unit 160 is information in which the defocus and the like are prevented or reduced, and therefore is presented as information helpful for the operator, such as the doctor, to use it for the diagnosis or the like.

In this manner, according to the present embodiment, the apparatus that irradiates the subject 100 with the spherical acoustic wave and acquires the subject information based on the acoustic wave reflected and propagated inside the subject 100 can acquire the subject information with high accuracy (high image quality) in which the defocus and the like are prevented or reduced.

A sixth embodiment will be described as an apparatus that irradiates the subject 100 with a focused acoustic wave, and acquires the subject information with use of a reception signal acquired by detecting the acoustic wave reflected and propagated inside the subject 100. Similar components to those of the first to fifth embodiments will be identified by the same reference numerals in principle, and descriptions thereof will be omitted.

<Configuration of Information Acquisition Apparatus>

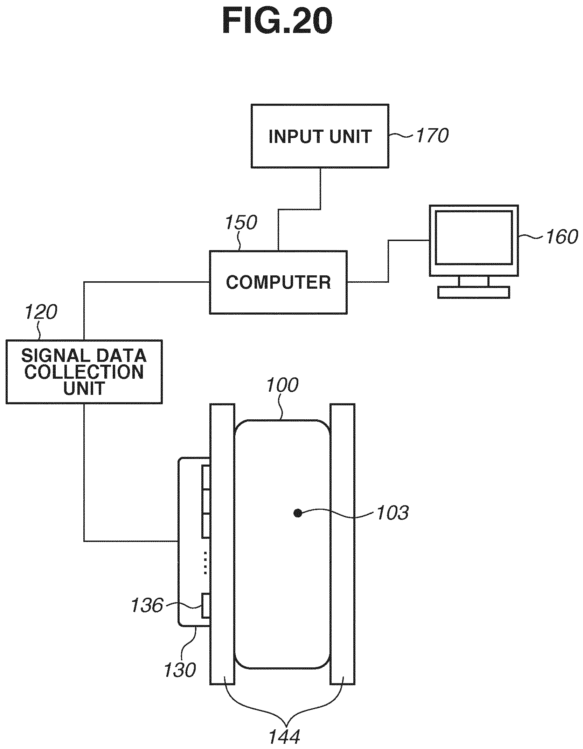

Fig. 20 is a schematic diagram of an information acquisition apparatus according to the present embodiment. Each of components of the apparatus will be described below. The apparatus includes the probe 130 including a transducer 136, the holding plates 144 as the holding unit, the computer 150, the display unit 160, and the input unit 170. The measurement target is the subject 100.

The probe 130 according to the present embodiment is configured in such a manner that a plurality of transducers 136 is lined along a straight line (1D) or arranged on a flat plane (2D). The transducers 136 do not have to be laid out in these manners, and may be laid out in any manner as long as they are laid out so as to be able to focus a beam when transmitting it.

The two holding plates 144 configured as parallel flat plates maintain the shape of the subject 100 by sandwiching the subject 100 therebetween.

<Method for Acquiring Subject Information>

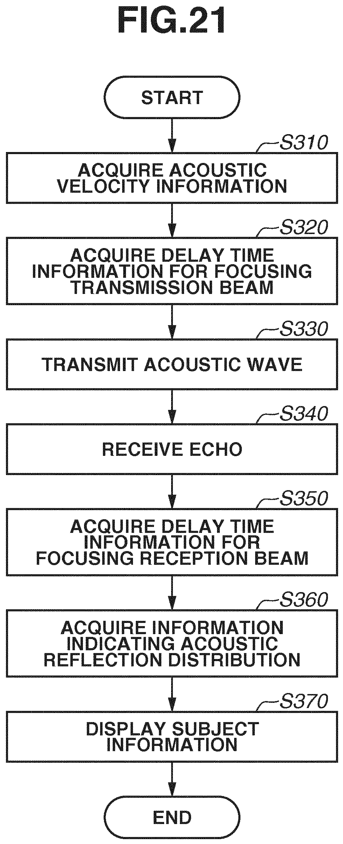

Next, each of processes in a method for acquiring the subject information according to the present embodiment will be described with reference to Fig. 21. The computer 150 controls the operation of each of the units of the information acquisition apparatus, by which each of the processes is performed.

<Step S310: Process for Acquiring Acoustic Velocity Information>

The computer 150 acquires the acoustic velocity information in the subject 100, the holding plate 144, the medium, and the like by a similar method to step S130.

<Step S320: Process for Calculating Delay Time for Focusing Transmission Beam>

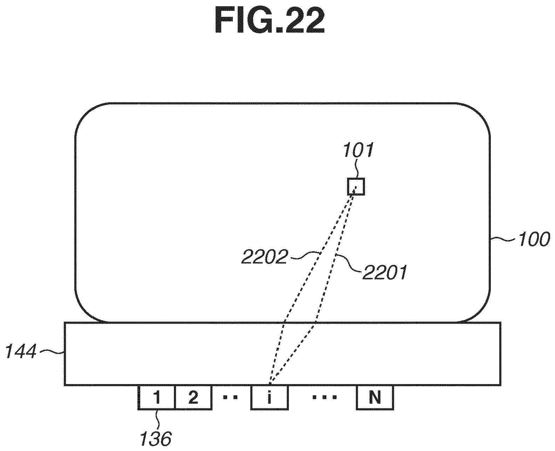

In the present process, the computer 150 calculates a delay time provided to each of the transducers 136 for transmitting the focused acoustic wave to the subject 100 (focusing a transmission beam). Fig. 22 illustrates an acoustic ray connecting the transducer 136 and the voxel 101 from which the subject information is acquired, to each other. An acoustic ray 2201 indicates a refracted acoustic ray of an i-th transducer 136 when the acoustic wave is propagated as the longitudinal wave in the holding plate 144.

First, the computer 150 calculates a propagation time $t_i^l$ regarding the acoustic ray 2201. The method described in the description of the fifth embodiment may be used for this calculation. The computer 150 calculates the propagation times $t_i^l$ regarding all of the transducers 136, and sets a longest propagation time among them as $t_b$. Next, the computer 150 calculates a transmission delay time $\tau_i$ of each of the transducers 136 with use of an equation (18).

$$\tau_i = t_b - t_i^l \quad (18)$$

<Step S330: Process for Irradiating Subject 100 with Acoustic Wave>

In the present process, the computer 150 drives each of the transducers 136 according to an equation (19), transmits the focused acoustic wave, and irradiates the subject 100 therewith. In the equation (19), $\tau_i$ represents the transmission delay time calculated in step S320.

$$s(r_i, t) = \delta(t - \tau_i) \quad (19)$$

The acoustic wave to be focused on the position of the voxel 101 can be generated by setting 0 as a time at which the transducer 136 having the longest propagation time $t_b$ is driven, and providing the transmission delay time $\tau_i$ to a signal for driving each of the transducers 136. In the equation (19), the driving signal issued to the transducer 136 is expressed as a delta function. In practice, it is difficult to generate the acoustic wave according to the delta function due to, for example, an impulse characteristic of the transducer 136, but the driving signal is expressed as the delta function for simplification of the description because this assumption does not impair the essence of the present invention.

<Step S340: Process for Receiving Echo>

In the present process, the probe 130 outputs an electric signal by receiving the acoustic wave (echo) reflected and propagated in the subject 100 after the subject 100 has been irradiated with the acoustic wave in step S330. The output electric signal is transferred to the computer 150.

<Step S350: Process for Acquiring Delay Time for Focusing Reception Beam>

In the present process, the computer 150 calculates a reception delay time for focusing a reception beam.

First, the computer 150 acquires the propagation time $t_i^l$ of the acoustic ray 2201. The value calculated in step S320 can be used therefor. Next, the computer 150 calculates a propagation time $t_i^t$ regarding an acoustic ray 2202. The acoustic ray 2202 indicates a refracted acoustic ray of the i-th transducer 136 when the acoustic wave is propagated as the transverse wave in the holding plate 144.

The computer 150 calculates two reception delay times $\rho_i^l$ and $\rho_i^t$ according to an equation (20) with use of the

propagation times $t_i^l$ and $t_i^t$ of the longitudinal wave and the transverse wave.

$$\rho_i^l \;=\; t_b \;+\; t_i^l$$

$$\rho_i^t \;=\; t_b \;+\; t_i^t \qquad (20)$$

<Step S360: Process for Acquiring Information indicating Acoustic Reflection Distribution>

In the present process, the computer 150 calculates the value $I(r_0)$ regarding the acoustic reflection at the voxel 101 based on the two reception delay times $\rho_i^l$ and $\rho_i^t$ calculated in step S350. An equation (21) is used for the calculation.

$$I(\mathbf{r}_0) = \sum_i^N \left[ p\!\left(\mathbf{r}_i, \rho_i^l\right) + p\!\left(\mathbf{r}_i, \rho_i^t\right) \right] \qquad (21)$$

In the equation (21), $r_0$ represents the positional vector of the voxel 101, $r_i$ represents the positional vector of the transducer 136, and N represents the number of transducers 136 for use in the reconstruction. The equation (21) indicates that the longitudinal wave signals and the transverse wave signals included in the reception signals $p(r_i, t)$ are subjected to delaying and summing. As a result, in addition to the longitudinal wave signals, the transverse wave signals are also correctly delayed and summed regarding the voxel 101, whereby the defocus can be prevented or reduced. The weights allocated to the longitudinal wave signal and the transverse wave signal may be changed as described in the description of the first embodiment. Further, the used signal may be any one of the longitudinal wave signal and the transverse wave signal as described in the description of the second embodiment.

In the case where there is a plurality of voxels 101 in the imaging target area of the subject 100, the information indicating the acoustic reflection distribution may be acquired by repeating steps S320 to S360 for each of these voxels 101. Alternatively, the information indicating the acoustic reflection distribution may be acquired by performing the processing in steps S350 and S360 for each of the plurality of voxels 101 contained in the area at which the transmitted acoustic wave is focused, with use of the signal acquired by transmitting the acoustic wave once (step S330). This method can shorten the measurement time by cutting down the number of times that the acoustic wave is transmitted. In this case, the longest propagation time $t_b$, the propagation time $t_i^l$ of the longitudinal wave, and the propagation time $t_i^t$ of the transverse wave are recalculated regarding the new voxel position $r_0$', and $I(r_0{}')$ is calculated based on the equation (20) and the equation (21).

<Step S370: Process for Displaying Subject Information>

In the present process, the information indicating the acoustic reflection distribution acquired in step S360 is displayed on the display unit 160 as the subject information of the imaging target area. The subject information displayed on the display unit 160 is information in which the defocus and the like are prevented or reduced, and therefore is presented as information helpful for the operator, such as the doctor, to use it for the diagnosis or the like.

In the above-described method, the propagation time $t_i^t$ of the transverse wave is taken into consideration only when the reception beam is focused, but may also be taken into consideration at the time of the transmission. For example, a transmission delay time $\tau_i$ expressed by an equation (22) is calculated with use of the method for calculating the propagation time t according to the second embodiment, and the focused acoustic wave is generated according to the equation (19).

$$\tau_i \;=\; t_b{}' \;-\; t_i^{a(i)} \qquad a(i) = \left\{ \begin{array}{l} l \\ t \end{array} \right. \qquad (22)$$

Regarding the transducer 136 for which the propagation time $t_i^l$ of the longitudinal wave is calculated, "l" is allocated to a(i). On the other hand, regarding the transducer 136 for which the propagation time $t_i^t$ of the transverse wave is calculated, "t" is allocated to a(i). In the equation (22), $t_b'$ represents a longest propagation time regarding $t_i^{a(i)}$ (i = 1 to N).

An equation (23) is used in the calculation of the reception delay time.

$$\rho_i = t_b' + t_i^{a(i)} \qquad (23)$$

An equation (24) is used in the calculation of the value $I(r_0)$ regarding the acoustic reflection.

$$I(\mathbf{r}_0) = \sum_i^N p(\mathbf{r}_i, \rho_i) \qquad (24)$$

This method can achieve beamforming while selecting a reception signal having a higher SN at the time of the transmission and the reception, and therefore can prevent or reduce the deterioration of the accuracy of the subject information.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to embodiments, it is to be understood that the invention is not limited to the disclosed embodiments. The following statements form part of the description and describe embodiments of the invention. The claims follow these statements and are labelled as such.

Statement 1. An information acquisition apparatus for processing a signal acquired by receiving an acoustic wave generated in a subject by irradiating the subject said acoustic wave being propagated through a medium provided between the subject and a reception means, the information acquisition apparatus comprising:

a position setting means for setting a position of interest; and
an acquisition means for determining a first signal of interest derived from an acoustic wave generated at the position of interest and propagated through the medium as a transverse wave, said first signal being determined using information indicating an acoustic velocity of the transverse wave in the medium, and acquiring subject information at the position of interest using the first signal of interest.

Statement 2. An information acquisition apparatus for processing a signal acquired by receiving an acoustic wave generated in a subject by irradiating the subject with a first acoustic wave said acoustic wave being propagated through a medium provided between the subject and a reception means, the information acquisition apparatus comprising:

a position setting means for setting a position of interest; and
an acquisition means for determining a first signal of interest derived from an acoustic wave generated at the position of interest and propagated through the medium as a transverse wave, said first signal being determined using information indicating an acoustic velocity of the transverse wave in the medium, and acquiring subject information at the position of interest using the first signal of interest.

Statement 3. A signal processing method comprising:

acquiring a signal derived from an acoustic wave generated in a subject by irradiating the subject and propagated through a medium provided between the subject and a reception means;

setting a position of interest;

determining a first signal of interest derived from the acoustic wave generated at the position of interest and propagated through the medium as a transverse wave, said first signal being determined with use of information indicating an acoustic velocity of the transverse wave in the medium; and

acquiring subject information at the position of interest using the first signal of interest.

Statement 4. A signal processing method comprising:

acquiring a signal derived from an acoustic wave generated in a subject by irradiating the subject with a first acoustic wave and propagated through a medium provided between the subject and a reception means;

setting a position of interest;

determining a first signal of interest derived from the acoustic wave generated at the position of interest and propagated through the medium as a transverse wave, said first signal being determined with use of information indicating an acoustic velocity of the transverse wave in the medium; and

acquiring subject information at the position of interest using the first signal of interest.

**Claims**

1. An information acquisition apparatus for processing a signal acquired by receiving an acoustic wave generated in a subject by irradiating the subject with light, said acoustic wave being propagated through a medium provided between the subject and a reception means, the information acquisition apparatus comprising:

a position setting means for setting a position of interest; and

an acquisition means for determining a first signal of interest derived from an acoustic wave generated at the position of interest and propagated through the medium as a transverse wave, said first signal being determined using information indicating an acoustic velocity of the transverse wave in the medium, and acquiring subject information at the position of interest using the first signal of interest.

2. The information acquisition apparatus according to claim 1, further comprising a mounting means for allowing a holding means as the medium to be mounted, the holding means being suitable for holding the subject.

3. The information acquisition apparatus according to claim 2, further comprising a storage means for storing a plurality of pieces of information each indicating the acoustic velocity of the transverse wave in the holding means, wherein the mounting means is configured to allow one of a plurality of holding means to be mounted each such holding means being suitable for holding the subject, and wherein the acquisition means is arranged to acquire the information indicating the acoustic velocity of the transverse wave in the holding means that corresponds to the holding means mounted on the mounting means by reading out the information from the storage means.

4. The information acquisition apparatus according to claim 1, wherein the acquisition means is arranged to acquire information indicating a first propagation time of the acoustic wave generated at the position of interest and propagated through the medium as the transverse wave with use of the information indicating the acoustic velocity of the transverse wave in the medium, and determine the first signal of interest with use of the information indicating the first propagation time.

5. The information acquisition apparatus according to any one of claims 1 to 4, wherein the acquisition means is arranged to determine a second signal of interest derived from the acoustic wave generated at the position of interest and propagated through the medium as a longitudinal wave with use of information indicating an acoustic velocity of the longitudinal wave in the medium, and determine the subject information at the position of interest with use of the first signal of interest and the second signal of interest.

6. The information acquisition apparatus according to claim 5, further comprising a determination means for determining a magnitude relationship between an incident angle at which the acoustic wave generated at the position of interest and to be received by the reception means is incident on the medium, and each of a critical angle and a hypothetical critical angle larger than this critical angle,

wherein the acquisition means is arranged to acquire the subject information at the position of interest with use of the first signal of interest and the second signal of interest, in a case where the incident angle is equal to or larger than the critical angle and smaller than the hypothetical critical angle.

7. The information acquisition apparatus according to claim 6, wherein the acquisition means is arranged to acquire the subject information at the position of interest with use of the second signal of interest without use of the first signal of interest, in a case where the incident angle is larger than the hypothetical critical angle.

8. The information acquisition apparatus according to claim 6 or 7, wherein the acquisition means is arranged to acquire the subject information at the position of interest with use of the first signal of interest without use of the second signal of interest, in a case where the incident angle is smaller than the critical angle.

9. The information acquisition apparatus according to claim 8, wherein the acquisition means is arranged to determine the first signal of interest assuming that the acoustic wave as the longitudinal wave is propagated in a direction normal to a surface of the medium, in a case where the incident angle is larger than the critical angle and smaller than the hypothetical critical angle, and determine the first signal of interest assuming that the acoustic wave as the longitudinal wave is refracted on the medium, in a case where the incident angle is smaller than the critical angle.

10. The information acquisition apparatus according to any one of claims 6 to 9, further comprising an angle setting means for setting the hypothetical critical angle,
wherein the angle setting means is arranged to set the hypothetical critical angle based on information indicating an instruction from a user,
wherein the acquisition means is arranged to cause the subject information acquired based on the hypothetical critical angle to be displayed on a display means, and
wherein the angle setting means is arranged to update the hypothetical critical angle based on the information indicating the instruction from the user that is received while the subject information is displayed on the display means.

11. The information acquisition apparatus according to any one of claims 5 to 10, wherein the acquisition means is arranged to weight the first signal of interest based on a pressure transmittance of the transverse wave through the medium, weight the second signal of interest based on a pressure transmittance of the longitudinal wave through the medium, and acquire the subject information at the position of interest with use of the weighted first signal of interest and the weighted second signal of interest.

12. The information acquisition apparatus according to any one of claims 5 to 10, wherein the acquisition means is arranged to weight the first signal of interest and the second signal of interest based on a weight determined based on an instruction from a user, acquire the subject information at the position of interest with use of the weighted first signal of interest and the weighted second signal of interest, and update the weight based on the information indicating the instruction from the user that is received while the subject information is displayed on the display means.

13. The information acquisition apparatus according to any one of claims 1 to 12, further comprising a light irradiation means for irradiating the subject with light,
wherein the reception means is arranged to output the signal by receiving the acoustic wave generated in the subject.

14. A signal processing method comprising:

acquiring a signal derived from an acoustic wave generated in a subject by irradiating the subject with light and propagated through a medium provided between the subject and a reception means;
setting a position of interest;
determining a first signal of interest derived from the acoustic wave generated at the position of interest and propagated through the medium as a transverse wave, said first signal being determined with use of information indicating an acoustic velocity of the transverse wave in the medium; and
acquiring subject information at the position of interest using the first signal of interest.

15. A program for causing a computer to perform the signal processing method according to claim 14.

# FIG.1

# FIG.2A

112  132

131

y

x

# FIG.2B

132

131

z

x

112

# FIG.2C

112  132

131

y

x

# FIG.2D

131

132

z

x

112

# FIG.3

# FIG.4

```
            ( START )
                │
                ▼            ┌─S110
    ┌───────────────────────┐
    │   IRRADIATE SUBJECT   │
    │     WITH LIGHT        │
    └───────────────────────┘
                │
                ▼            ┌─S120
    ┌───────────────────────┐
    │       RECEIVE         │
    │  PHOTOACOUSTIC WAVE   │
    └───────────────────────┘
                │
                ▼            ┌─S130
    ┌───────────────────────┐
    │   ACQUIRE ACOUSTIC    │
    │  VELOCITY INFORMATION │
    └───────────────────────┘
                │
                ▼            ┌─S140
    ┌───────────────────────┐
    │  ACQUIRE INFORMATION  │
    │ INDICATING PROPAGATION│
    │ TIME OF ACOUSTIC WAVE │
    └───────────────────────┘
                │
                ▼            ┌─S150
    ┌───────────────────────┐
    │   ACQUIRE SUBJECT     │
    │     INFORMATION       │
    └───────────────────────┘
                │
                ▼            ┌─S160
    ┌───────────────────────┐
    │   DISPLAY SUBJECT     │
    │     INFORMATION       │
    └───────────────────────┘
                │
                ▼
            (  END  )
```

# FIG.5A

# FIG.5B

# FIG.5C

# FIG.5D

# FIG.5E

# FIG.6A

# FIG.6B

# FIG.7

# FIG.8

# FIG.9

# FIG.10A

# FIG.10B

# FIG.11A

# FIG.11B

# FIG.12

# FIG.13A

# FIG.13B

# FIG.14

$100$ $\left[ c_1 = 1510\ [\text{m/s}] \right]$

0.5 mm

ACOUSTIC
SOURCE

$140$ $\left[ \begin{array}{l} c_2^l = 2340\ [\text{m/s}] \\ c_2^t = 912\ [\text{m/s}] \end{array} \right]$

$c_3$

$\left[ c_3 = 1480\ [\text{m/s}] \right]$

$132$

$131$

$130$

z
y
x

# FIG.15

IMAGE
INTENSITY

*A*

*B*

X AXIS DIRECTION

# FIG.16A

| PROPAGATION TIME OF LONGITUDINAL WAVE | | | | |
|---|---|---|---|---|
| | $\cdots$ | i | i+1 | $\cdots$ |
| $\vdots$ | | | | |
| j | | $t^l(i, j)$ | $t^l(i+1, j)$ | |
| $\vdots$ | | | | |

| PROPAGATION TIME OF TRANSVERSE WAVE | | | | |
|---|---|---|---|---|
| | $\cdots$ | i | i+1 | $\cdots$ |
| $\vdots$ | | | | |
| j | | $t^t(i, j)$ | $t^t(i+1, j)$ | |
| $\vdots$ | | | | |

# FIG.16B

*101* j-TH VOXEL

$t^l(i+1, j)$

$t^l(i, j)$

*131* i+1-TH TRANSDUCER

*131* i-TH TRANSDUCER

# FIG.16C

| | $\cdots$ | i | i+1 | $\cdots$ |
|---|---|---|---|---|
| $\vdots$ | | | | |
| j | | $t^l(i, j)$ | $t^t(i+1, j)$ | |
| $\vdots$ | | | | |

44

# FIG.17

INPUT UNIT ~170

150

COMPUTER

~160

120

SIGNAL DATA
COLLECTION
UNIT

100

103

141

142    141

143

140

140

132

134

130

135

# FIG.18

START

↓ ─S210

IRRADIATE SUBJECT
WITH ACOUSTIC WAVE

↓ ─S220

RECEIVE ECHO

↓ ─S230

ACQUIRE ACOUSTIC
VELOCITY INFORMATION

↓ ─S240

ACQUIRE INFORMATION
INDICATING PROPAGATION
TIME OF ACOUSTIC WAVE

↓ ─S250

ACQUIRE SUBJECT
INFORMATION

↓ ─S260

DISPLAY SUBJECT
INFORMATION

↓

END

# FIG.19

# FIG.20

INPUT UNIT ~ *170*

*150*

COMPUTER ~ *160*

*120*

SIGNAL DATA
COLLECTION
UNIT

*100*

~ *103*

*136*

*130*

*144*

# FIG.21

START

↓ S310

ACQUIRE ACOUSTIC
VELOCITY INFORMATION

↓ S320

ACQUIRE DELAY TIME
INFORMATION FOR FOCUSING
TRANSMISSION BEAM

↓ S330

TRANSMIT ACOUSTIC WAVE

↓ S340

RECEIVE ECHO

↓ S350

ACQUIRE DELAY TIME
INFORMATION FOR
FOCUSING RECEPTION BEAM

↓ S360

ACQUIRE INFORMATION
INDICATING ACOUSTIC
REFLECTION DISTRIBUTION

↓ S370

DISPLAY SUBJECT
INFORMATION

↓

END

# FIG.22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 6607489 B